# EUROPEAN PATENT APPLICATION

(11) **EP 4 506 363 A1**
(43) Date of publication of application: **12.02.2025**
(21) Application number: 23778456.6
(22) Date of filing: 31.03.2023
(51) Int. Cl.: C07K 16/28, C07K 16/46, C12N 15/13, A61K 39/395, A61P 35/00

(54) **MONOCLONAL ANTIBODY AND BISPECIFIC ANTIBODY AGAINST C-MET**

(30) Priority: 02.04.2022 CN 202210343364; 02.04.2022 CN 202210345737
(71) Applicant: Biotheus Inc., Tangjiawan Town, Xiangzhou District Zhuhai, Guangdong 519080 (CN)
(72) Inventor: LUO, Yi, Zhuhai, Guangdong 519080 (CN); MIAO, Xiaoniu, Zhuhai, Guangdong 519080 (CN); YAN, Yao, Zhuhai, Guangdong 519080 (CN); WANG, Chao, Zhuhai, Guangdong 519080 (CN); XU, Yingda, Zhuhai, Guangdong 519080 (CN); WANG, Ping, Zhuhai, Guangdong 519080 (CN); ZHANG, Jie, Zhuhai, Guangdong 519080 (CN); ZHAO, Jiaoyang, Zhuhai, Guangdong 519080 (CN)
(74) Representative: Cabinet Nony
(86) International application number: PCT/CN2023/085460
(87) International publication number: WO 2023/186092

(57) **Abstract**

The present application relates to an antibody capable of specifically binding to c-Met or antigen-binding fragment thereof, and an immunoconjugate, a pharmaceutical composition and a multispecific molecule comprising the same. The present application further relates to the use of the antibody specifically binding to c-Met or an antigen-binding fragment thereof and the multispecific molecule. Compared with the control antibody, the bispecific antibody or defucosylated bispecific antibody of the present application can block HGF-dependent TKI resistance; block the proliferation and migration of tumor cells induced by HGF, induce ADCC effect, and inhibit tumor growth in vivo.

## Description

### Technical Field

The present application belongs to the field of biomedical technology. More specifically, the present application relates to an antibody or antigen-binding fragment thereof capable of specifically binding to c-Met, as well as an immunoconjugate, pharmaceutical composition and multispecific molecule comprising the antibody or antigen-binding fragment thereof. Furthermore, the present application also relates to the use of the antibody or antigen-binding fragment thereof capable of specifically binding to c-Met as well as the multispecific molecule.

### Background

The c-Met protein is a receptor tyrosine kinase, which is converted from a 170kDa leader protein into an α subunit of 50kDa and a β subunit of 145kDa through post-translational modification. It forms a transmembrane dimer after disulfide bond connection. At present, the main known ligand of c-Met is hepatocyte growth factor (HGF). Upon receipt of HGF stimulation, the intracellular domain of c-Met undergoes auto-phosphorylation at tyrosine residues Y1234 and Y1235, and then the phosphorylation signal is transduced to Y1349 and Y1356, enabling the intracellular domain of c-Met bind to the adaptor proteins. The downstream signal activation pathways of c-Met include PI3K/Akt, Rac1/Cdc42, and Erk/MAPK, which can significantly affect relevant manifestations such as cell proliferation, migration, infiltration, and tube tissue formation. After the c-Met protein is activated by autophosphorylation, the Cb1 ubiquitin ligase initiates the ubiquitination of this protein, which then enters the degradation process, thereby performs negative regulation on the c-Met pathways.

Studies have shown that c-Met can promote cancer progression and is highly expressed in a variety of tumor tissues. This makes it a target for the development of anticancer drugs. In addition to traditional RTK small molecule inhibitors, the drug development of large molecule targeting antibodies has gradually advanced and deepened into clinical research. The example thereof includes the anti-c-Met monoclonal antibody MetMab. Other examples are ADC molecules such as TR1801, which is conjugated with the antitubulin toxin tesirine, is effective in mouse tumor PDX models with low, medium, and high abundance expression of MET.

Therefore, it is necessary to develop a new anti-c-Met monoclonal antibody and a multi-specific antibody containing it to have higher application potential in tumor treatment.

### Contents of the Invention

In the present application, an antibody or antigen-binding fragment thereof capable of specifically binding to c-Met is obtained, from this, a bispecific antibody capable of specifically binding to c-Met and EGFR is prepared. Furthermore, a afucosylated bispecific antibody is prepared and the present invention is completed.

In the first aspect, the present application provides an antibody or antigen-binding fragment thereof capable of specifically binding to c-Met, wherein the antibody or antigen-binding fragment thereof comprises:
(a) a heavy chain variable region (VH) comprising the following 3 complementary determining regions (CDRs):
   (i) VH CDR1, which is composed of the sequence as set forth in SEQ ID NO: 27 or 33, or a sequence having a substitution, deletion or addition of one or several amino acids (e.g., a substitution, deletion or addition of 1, 2 or 3 amino acids) as compared thereto,
   (ii) VH CDR2, which is composed of the sequence as set forth in SEQ ID NO: 28 or 34, or a sequence having a substitution, deletion or addition of one or several amino acids (e.g., a substitution, deletion or addition of 1, 2 or 3 amino acids) as compared thereto, and
   (iii) VH CDR3, which is composed of the sequence as set forth in any one of SEQ ID NO: 29 or 35, or a sequence having a substitution, deletion or addition of one or several amino acids (e.g., a substitution, deletion or addition of 1, 2 or 3 amino acids) as compared thereto;
   and/or,
(b) a light chain variable region (VL) comprising the following 3 complementarity determining regions (CDRs):
   (iv) VL CDR1, which is composed of the following sequence: SEQ ID NO: 30 or 36, or a sequence having a substitution, deletion or addition of one or several amino acids (e.g., a substitution, deletion or addition of 1, 2 or 3 amino acids) as compared thereto,
   (v) VL CDR2, which is composed of the following sequence: SEQ ID NO: 31 or 37, or a sequence having a substitution, deletion or addition of one or several amino acids (e.g., a substitution, deletion or addition of 1, 2 or 3 amino acids) as compared thereto, and
   (vi) VL CDR3, which is composed of the following sequence: SEQ ID NO: 32 or 38, or a sequence having a substitution, deletion or addition of one or several amino acids (e.g., a substitution, deletion or addition of 1, 2 or 3 amino acids) as compared thereto.

In certain embodiments, the substitution described in any one of (i) to (vi) is a conservative substitution.

In certain embodiments, the CDRs described in any one of (i) to (vi) are defined according to the Kabat, IMGT or Chothia numbering systems.

In certain embodiments, the CDRs described in any one of (i) to (vi) are defined according to the IMGT numbering system.

In certain embodiments, the antibody or antigen-binding fragment thereof as described above comprises: the following 3 heavy chain CDRs: VH CDR1 as set forth in SEQ ID NO: 27, VH CDR2 as set forth in SEQ ID NO: 28, VH CDR3 as set forth in SEQ ID NO: 29; and/or, the following 3 light chain CDRs: VL CDR1 as set forth in SEQ ID NO: 30, VL CDR2 as set forth in SEQ ID NO: 31, VL CDR3 as set forth in SEQ ID NO: 32.

In certain embodiments, the antibody or antigen-binding fragment thereof as described above comprises: the following 3 heavy chain CDRs: VH CDR1 as set forth in SEQ ID NO: 33, VH CDR2 as set forth in SEQ ID NO: 34, VH CDR3 as set forth in SEQ ID NO: 35; and/or, the following 3 light chain CDRs: VL CDR1 as set forth in SEQ ID NO: 36, VL CDR2 as set forth in SEQ ID NO: 37, VL CDR3 as set forth in SEQ ID NO: 38.

In certain embodiments, the antibody or antigen-binding fragment thereof further comprises a framework region of a human immunoglobulin.

In certain embodiments, the antibody or antigen-binding fragment thereof as described above comprises:
(a) a heavy chain variable region (VH), which comprises an amino acid sequence selected from the following:
   (i) the sequence as set forth in SEQ ID NO: 9 or 13;
   (ii) a sequence having a substitution, deletion or addition of one or several amino acids (e.g., a substitution, deletion or addition of 1, 2, 3, 4 or 5 amino acids) as compared to the sequence as set forth in SEQ ID NO: 9 or 13; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 9 or 13;
   and/or
(b) a light chain variable region (VL), which comprises an amino acid sequence selected from the following:
   (iv) the sequence as set forth in SEQ ID NO: 11 or 15;
   (v) a sequence having a substitution, deletion or addition of one or several amino acids (e.g., a substitution, deletion or addition of 1, 2, 3, 4 or 5 amino acids) as compared to the sequence as set forth in SEQ ID NO: 11 or 15; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 11 or 15.

In certain embodiments, the substitution described in (ii) or (v) is a conservative substitution.

In certain embodiments, the antibody or antigen-binding fragment thereof comprises: a VH having a sequence as set forth in SEQ ID NO: 9 and a VL having a sequence as set forth in SEQ ID NO: 11.

In certain embodiments, the antibody or antigen-binding fragment thereof comprises: a VH having a sequence as set forth in SEQ ID NO: 13 and a VL having a sequence as set forth in SEQ ID NO: 15.

In certain embodiments, the antibody or antigen-binding fragment thereof as described above, wherein it further comprises a constant region derived from a human immunoglobulin.

In certain embodiments, the heavy chain of the antibody or antigen-binding fragment thereof comprises a heavy chain constant region derived from a human immunoglobulin (e.g., IgG1, IgG2, IgG3, or IgG4).

In certain embodiments, the heavy chain constant region has a sequence as set forth in SEQ ID NO: 19, 20, 39, or 40.

In certain embodiments, the light chain of the antibody or antigen-binding fragment thereof comprises a light chain constant region derived from a human immunoglobulin (e.g., κ or λ).

In certain embodiments, the light chain constant region has a sequence as set forth in SEQ ID NO: 21, 22, or 41.

In certain embodiments, the antibody or antigen-binding fragment thereof has ADCC activity.

In certain embodiments, the antibody or antigen-binding fragment thereof comprises a mutated or chemically modified Fc region.

In certain embodiments, the antibody or antigen-binding fragment thereof comprises an Fc region having a LALA mutation and/or a knob-into-hole modification.

In certain embodiments, the antibody or antigen-binding fragment thereof comprises:
(1) a heavy chain having a sequence as set forth in SEQ ID NO: 10 and a light chain having a sequence as set forth in SEQ ID NO: 12;
(2) a heavy chain having a sequence as set forth in SEQ ID NO: 14 and a light chain having a sequence as set forth in SEQ ID NO: 16; or
(3) a heavy chain having a sequence as set forth in SEQ ID NO: 23 and a light chain having a sequence as set forth in SEQ ID NO: 24.

The related technology of "knob-into-hole" is described in, for example, US 5,731,168; US 7,695,936; Ridgway et al., Prot Eng 9, 617-621 (1996), and Carter, J Immunol Meth 248, 7-15 (2001).

In certain embodiments, the Fc region has an amino acid sequence as set forth in SEQ ID NO: 17 or 18.

In certain embodiments, the antibody or antigen-binding fragment thereof is hypofucosylated or afucosylated.

In certain embodiments, the antibody or antigen-binding fragment thereof as described above is selected from Fab, Fab', (Fab')², Fv, disulfide-linked Fv, scFv, diabody and single-domain antibody (sdAb); and/or, the antibody is a murine antibody, a chimeric antibody, a humanized antibody or a multispecific antibody.

On the other hand, the present application provides an isolated nucleic acid molecule encoding the antibody or antigen-binding fragment thereof as described above.

On the other hand, the present application provides a vector comprising the nucleic acid molecule as described above. In certain embodiments, the vector is a cloning vector or an expression vector.

On the other hand, the present application provides a host cell comprising the nucleic acid molecule as described above or the vector as described above.

In certain embodiments, the host cell is a mammalian cell.

In certain embodiments, the host cell has low or no fucosylation activity, for example, the host cell is selected from mammalian cells (e.g., CHO cells) lacking expression of a gene encoding a fucosyltransferase.

On the other hand, the present application provides a method for preparing the antibody or antigen-binding fragment thereof as described above, comprising culturing the host cell as described above under a condition that allows expression of the antibody or antigen-binding fragment thereof, and recovering the antibody or antigen-binding fragment thereof from a culture of the cultured host cell.

In certain embodiments, the host cell has low or no fucosylation activity, for example, the host cell is selected from mammalian cells (e.g., CHO cells) lacking expression of a gene encoding a fucosyltransferase.

On the other hand, the present application provides a multispecific molecule comprising the antibody or antigen-binding fragment thereof as described above.

**In** certain embodiments, the multispecific molecule specifically binds to c-Met, and additionally specifically binds to one or more other targets.

In certain embodiments, the multispecific molecule is a bispecific molecule.

In certain embodiments, the bispecific molecule further comprises a molecule (e.g., a second antibody) having a second binding specificity for a second target.

In certain embodiments, the second target is an epidermal growth factor receptor (EGFR), and the second antibody is an anti-EGFR antibody or antigen-binding fragment thereof.

In certain embodiments, the bispecific molecule has been modified by glycoslyation to have a lower number of fucose than the same bispecific molecule that has not been modified by glycoslyation.

In certain embodiments, the anti-EGFR antibody or antigen-binding fragment thereof is hypofucosylated or afucosylated.

In certain embodiments, the antibody or antigen-binding fragment thereof as described above is hypofucosylated or afucosylated.

In certain embodiments, the second antibody comprises: the following 3 heavy chain CDRs: VH CDR1 as set forth in SEQ ID NO: 46, VH CDR2 as set forth in SEQ ID NO: 47, VH CDR3 as set forth in SEQ ID NO: 48; and, the following 3 light chain CDRs: VL CDR1 as set forth in SEQ ID NO: 49, VL CDR2 as set forth in SEQ ID NO: 50, VL CDR3 as set forth in SEQ ID NO: 51.

In certain embodiments, the second antibody comprises:
(1) a VH having a sequence as set forth in SEQ ID NO: 1 and a VL having a sequence as set forth in SEQ ID NO: 3; or
(2) a VH having a sequence as set forth in SEQ ID NO: 52 and a VL having a sequence as set forth in SEQ ID NO: 53.

In certain embodiments, the second antibody comprises:
(1) a heavy chain having a sequence as set forth in SEQ ID NO: 2 and a light chain having a sequence as set forth in SEQ ID NO: 4; or
(2) a heavy chain having a sequence as set forth in SEQ ID NO: 25 and a light chain having a sequence as set forth in SEQ ID NO: 26.

In certain embodiments, the bispecific molecule comprises:
(1) a first antibody comprising VH CDRs 1-3 as set forth in SEQ ID NOs: 27-29 and VL CDRs 1-3 as set forth in SEQ ID NOs: 30-32; and, a second antibody comprising VH CDRs 1-3 as set forth in SEQ ID NOs: 46-48 and VL CDRs 1-3 as set forth in SEQ ID NOs: 49-51; or
(2) a first antibody comprising VH CDRs 1-3 as set forth in SEQ ID NOs: 33-35 and VL CDRs 1-3 as set forth in SEQ ID NOs: 36-38; and, a second antibody comprising VH CDRs 1-3 as set forth in SEQ ID NOs: 46-48 and VL CDRs 1-3 as set forth in SEQ ID NOs: 49-51.

In certain embodiments, the multispecific molecule comprises:
(1) a first antibody comprising a VH having a sequence as set forth in SEQ ID NO: 9 and a VL having a sequence as set forth in SEQ ID NO: 11; and, a second antibody comprising a VH having a sequence as set forth in SEQ ID NO: 1 and a VL having a sequence as set forth in SEQ ID NO: 3;
(2) a first antibody comprising a VH having a sequence as set forth in SEQ ID NO: 9 and a VL having a sequence as set forth in SEQ ID NO: 11; and, a second antibody comprising a VH having a sequence as set forth in SEQ ID NO: 52 and a VL having a sequence as set forth in SEQ ID NO: 53;
(3) a first antibody comprising a VH having a sequence as set forth in SEQ ID NO: 13 and a VL having a sequence as set forth in SEQ ID NO: 15; and, a second antibody comprising a VH having a sequence as set forth in SEQ ID NO: 1 and a VL having a sequence as set forth in SEQ ID NO: 3; or
(4) a first antibody comprising a VH having a sequence as set forth in SEQ ID NO: 13 and a VL having a sequence as set forth in SEQ ID NO: 15; and, a second antibody comprising a VH having a sequence as set forth in SEQ ID NO: 52 and a VL with a sequence as set forth in SEQ ID NO: 53.

In certain embodiments, the multispecific molecule comprises:
(1) a first antibody comprising a heavy chain having a sequence as set forth in SEQ ID NO: 10 and a light chain having a sequence as set forth in SEQ ID NO: 12; and, a second antibody comprising a heavy chain having a sequence as set forth in SEQ ID NO: 25 and a light chain having a sequence as set forth in SEQ ID NO: 26;
(2) a first antibody comprising a heavy chain having a sequence as set forth in SEQ ID NO: 10 and a light chain having a sequence as set forth in SEQ ID NO: 12; and, a second antibody comprising a heavy chain having a sequence as set forth in SEQ ID NO: 2 and a light chain having a sequence as set forth in SEQ ID NO: 4;
(3) a first antibody comprising a heavy chain having a sequence as set forth in SEQ ID NO: 14 and a light chain having a sequence as set forth in SEQ ID NO: 16; and, a second antibody comprising a heavy chain having a sequence as set forth in SEQ ID NO: 25 and a light chain having a sequence as set forth in SEQ ID NO: 26;
(4) a first antibody comprising a heavy chain having a sequence as set forth in SEQ ID NO: 14 and a light chain having a sequence as set forth in SEQ ID NO: 16; and, a second antibody comprising a heavy chain of the sequence as set forth in SEQ ID NO: 2 and a light chain of the sequence as set forth in SEQ ID NO: 4; or
(5) a first antibody comprising a heavy chain of the sequence as set forth in SEQ ID NO: 23 and a light chain of the sequence as set forth in SEQ ID NO: 24; and, a second antibody comprising a heavy chain of the sequence as set forth in SEQ ID NO: 25 and a light chain of the sequence as set forth in SEQ ID NO: 26.

On the other hand, there is provided a method for preparing the multispecific antibody as described above, comprising obtaining the antibody or antigen-binding fragment thereof as described above by the method as described above, contacting it with an anti-EGFR antibody or antigen-binding fragment thereof; optionally, contacting it with a reducing agent (e.g., DTT).

On the other hand, there is provided an immunoconjugate, comprising the antibody or antigen-binding fragment thereof as described above or the multispecific molecule as described above, and a therapeutic agent linked to the antibody or antigen-binding fragment thereof or the multispecific molecule.

In certain embodiments, the therapeutic agent is selected from a cytotoxic agent.

In certain embodiments, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof.

In certain embodiments, the immunoconjugate is an antibody-drug conjugate (ADC).

In another aspect, there is provided a pharmaceutical composition, comprising the antibody or antigen-binding fragment thereof as described above, or the multispecific molecule as described above, and a pharmaceutically acceptable carrier and/or excipient.

In certain embodiments, the pharmaceutical composition further comprises an additional pharmaceutically active agent.

In certain embodiments, the pharmaceutical composition further comprises an EGFR inhibitor.

In certain embodiments, the EGFR inhibitor is selected from the group consisting of erlotinib, gefitinib, osimertinib, or any combination thereof.

In certain embodiments, the EGFR inhibitor and the antibody or antigen-binding fragment thereof, or the multispecific molecule are respectively contained in different preparations as active components and are administered simultaneously or at different times. In certain embodiments, the EGFR inhibitor is osimertinib.

In certain embodiments, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

On the other hand, there is provided a use of the antibody or antigen-binding fragment thereof as described above, or the multispecific molecule as described above in combination with an EGFR inhibitor in manufacture of a medicament.

In certain embodiments, the EGFR inhibitor is selected from the group consisting of erlotinib, gefitinib, osimertinib, or any combination thereof; in certain embodiments, the EGFR inhibitor is osimertinib.

In certain embodiments, the medicament is used for:
(1) increasing immune cell activity in vitro or in vivo in a subject;
(2) enhancing an immune response in a subject;
(3) preventing and/or treating a tumor in a subject; or
(4) preventing and/or treating an infection in a subject;

In certain embodiments, the tumor expresses c-Met.

In certain embodiments, the tumor involves a tumor cell expressing c-Met. In certain embodiments, the c-Met is expressed on the surface of the tumor cell.

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In certain embodiments, the infection is selected from the group consisting of viral infection, bacterial infection, fungal infection and parasitic infection.

In certain embodiments, the subject is a mammal, such as a human, a cynomolgus monkey or a mouse.

In another aspect, there is provided a kit, which comprises the antibody or antigen-binding fragment thereof as described above.

In some embodiments, the antibody or antigen-binding fragment thereof comprises a detectable label, such as an enzyme (e.g., horseradish peroxidase), a radionuclide, a fluorescent dye, a luminescent substance (e.g., a chemiluminescent substance), or biotin.

In some embodiments, the kit further comprises a second antibody that specifically recognizes an anti-EGFR antibody or antigen-binding fragment thereof.

In some embodiments, the second antibody further comprises a detectable label, such as an enzyme (e.g., horseradish peroxidase), a radionuclide, a fluorescent dye, a luminescent substance (e.g., a chemiluminescent substance), or biotin.

In some embodiments, the anti-EGFR antibody or antigen-binding fragment thereof is hypofucosylated or afucosylated.

In some embodiments, the antibody or antigen-binding fragment thereof as described above is hypofucosylated or afucosylated.

In another aspect, there is provided a chimeric antigen receptor, which comprises an antigen-binding domain of the antibody or antigen-binding fragment thereof as described above.

In some embodiments, the antigen-binding domain comprises a heavy chain variable region and a light chain variable region of the antibody or antigen-binding fragment thereof as described above.

In certain embodiments, the chimeric antigen receptor is expressed by an immune effector cell (e.g., a T cell).

In another aspect, there is provided a method for inhibiting the growth of a tumor cell expressing c-Met and/or killing the tumor cell, comprising contacting the tumor cell with an effective amount of the antibody or antigen-binding fragment thereof as described above, or the multispecific molecule as described above, or the immunoconjugate as described above, or the pharmaceutical composition as described above, or the chimeric antigen receptor as described above.

In another aspect, there is provided a use of the antibody or antigen-binding fragment thereof as described above, or the multispecific molecule as described above, or the immunoconjugate as described above, or the pharmaceutical composition as described above, or the chimeric antigen receptor as described above in the manufacture of a medicament, wherein the medicament is used for:
(1) increasing immune cell activity in vitro or in vivo in a subject;
(2) enhancing an immune response in a subject;
(3) preventing and/or treating a tumor in a subject; or
(4) preventing and/or treating an infection in a subject.

In certain embodiments, the tumor expresses c-Met.

In certain embodiments, the tumor involves a tumor cell expressing c-Met. In certain embodiments, the c-Met is expressed on the surface of the tumor cell.

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In some embodiments, the infection is selected from the group consisting of viral infection, bacterial infection, fungal infection and parasitic infection.

In some embodiments, the subject is a mammal, such as a human, a cynomolgus monkey or a mouse.

In another aspect, there is provided a use of the antibody or antigen-binding fragment thereof as described above in the manufacture of a kit, wherein the kit is used for determining whether a tumor can be treated by an anti-tumor therapy targeting c-Met;
(1) contacting a sample containing the tumor cell with the antibody or antigen-binding fragment thereof as described above;
(2) detecting the formation of a complex comprising the antibody or antigen-binding fragment thereof and c-Met.

In some embodiments, the antibody or antigen-binding fragment thereof comprises a detectable label.

In some embodiments, the c-Met is c-Met of a mammal (e.g., a human, a monkey).

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In one aspect, the present application provides a method for preventing and/or treating a tumor in a subject, the method comprising administering to a subject in need thereof an effective amount of the antibody or antigen-binding fragment thereof as described above, or the bispecific or multispecific molecule as described above, or the immunoconjugate as described above, or the pharmaceutical composition as described above, or the chimeric antigen receptor as described above, or the host cell as described above.

In certain embodiments, the tumor expresses c-Met.

In certain embodiments, the tumor involves a tumor cell expressing c-Met. In certain embodiments, the c-Met is expressed on the surface of the tumor cell.

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In certain embodiments, the subject is a mammal, such as a human.

In certain embodiments, the method further comprises administering an additional drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the method further comprises administering an additional anti-tumor therapy, such as a surgery, a chemotherapy, radiotherapy, a targeted therapy, an immunotherapy, a hormone therapy, a gene therapy, or a palliative care.

On the other hand, the present application provides a method for determining whether a tumor can be treated by an anti-tumor therapy targeting c-Met, which comprises the following steps:
(1) contacting a sample containing the tumor cell with the antibody or antigen-binding fragment thereof as described above;
(2) detecting the formation of a complex comprising the antibody or antigen-binding fragment thereof and c-Met.

In certain embodiments, the antibody or antigen-binding fragment thereof comprises a detectable label.

In certain embodiments, the c-Met is c-Met of a mammalian (e.g., a human, a monkey).

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

On the other hand, the present application provides a method for detecting the presence or amount of c-Met in a sample, which comprises the following steps:
(1) contacting the sample with the antibody or antigen-binding fragment thereof as described above;
(2) detecting the formation of a complex comprising the antibody or antigen-binding fragment thereof and c-Met or detecting the amount of the complex.

In certain embodiments, the antibody or antigen-binding fragment thereof comprises a detectable label.

In certain embodiments, the c-Met is c-Met of a mammalian (e.g., a human, a monkey).

### Definition of terms

In the present invention, unless otherwise specified, the scientific and technical terms used herein have the meanings commonly understood by those skilled in the art. In addition, the operation steps of molecular genetics, nucleic acid chemistry, chemistry, molecular biology, biochemistry, cell culture, microbiology, cell biology, genomics and recombinant DNA used herein are all conventional steps widely used in the corresponding fields. At the same time, in order to better understand the present invention, the definitions and explanations of relevant terms are provided below.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotypes as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissue or factor, including the binding of various cells of the immune system (e.g., effector cells) to the first component of the classical complement system (C1q). The VH and VL regions can also be subdivided into regions of high variability called complementarity determining regions (CDRs), interspersed with more conservative regions called framework regions (FRs). Each VH and VL consists of 3 CDRs and 4 FRs arranged from the amino terminus to the carboxyl terminus in the following sequence: FR1, CDR1, FR2, CDR2, FR3, CDR3, FR4. The variable regions (VH and VL) of each heavy chain/light chain pair respectively form an antigen-binding site. The assignment of amino acids to regions or domains can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to those amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia or IMGT numbering systems.

As used herein, the term "framework region" or "FR" residues refers to those amino acid residues in the variable region of an antibody other than the CDR residues defined above.

The term "antibody" is not limited to any particular method of producing the antibody. For example, it includes recombinant antibody, monoclonal antibody and polyclonal antibody. The antibody can be an antibody of different isotypes, for example, IgG (e.g., IgG1, IgG2, IgG3 or IgG4 subtype), IgA1, IgA2, IgD, IgE or IgM antibody.

As used herein, the terms "monoclonal antibody", "McAb", "mAb" have the same meaning and are used interchangeably, and refer to an antibody or a fragment of an antibody derived from a group of highly homologous antibody molecules, that is, a group of identical antibody molecules except for natural mutations that may occur spontaneously. Monoclonal antibody has high specificity for a single epitope on an antigen. Polyclonal antibody is mentioned relatively to monoclonal antibody, which usually contains at least 2 or more different antibodies, which usually recognize different epitopes on an antigen. In addition, the modifier "monoclonal" only indicates that the antibody is characterized by being obtained from a highly homologous antibody group, and it should not be interpreted that the antibody needs to be prepared by any specific method.

The monoclonal antibody of the present invention can be prepared by a variety of techniques, such as hybridoma technology (see, for example, Kohler et al., Nature, 256:495, 1975), recombinant DNA technology (see, for example, U.S. Patent Application 4,816,567), or phage antibody library technology (see, for example, Clackson et al. Nature 352: 624-628, 1991, or Marks et al. J. Mol. Biol. 222: 581-597, 1991).

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of an antibody can be obtained by recombinant DNA technology or by enzymatic or chemical cleavage of an intact antibody. Non-limiting examples of antigen-binding fragments include Fab, Fab', F(ab')², Fd, Fv, complementarity determining region (CDR) fragment, scFv, diabody, single domain antibody, chimeric antibody, linear antibody, nanobody (technology from Domantis), probody, and such polypeptides, which contain at least a portion of an antibody that is sufficient to confer specificity to the polypeptides with antigen-binding capability. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains." Among them, "full-length heavy chain" refers to a polypeptide chain that consists of a heavy chain variable region (VH), a heavy chain constant region CH1 domain, a hinge region (HR), a heavy chain constant region CH2 domain, a heavy chain constant region CH3 domain in the direction from the N-terminal to the C-terminal; and, when the full-length antibody is of IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and disulfide bonds between the HRs of the two full-length heavy chains. The full-length antibody of the present invention may be from a single species, such as human; and it may also be a chimeric antibody or humanized antibody. The full-length antibody of the present invention comprises two antigen binding sites formed by VH and VL pairs, respectively, which specifically recognize/bind to the same antigen.

As used herein, the term "Fd" refers to an antibody fragment consisting of VH and CH1 domains; the term "dAb fragment" refers to an antibody fragment consisting of VH domain (Ward et al., Nature 341:544 546 (1989)); the term "Fab fragment" refers to an antibody fragment consisting of VL, VH, CL and CH1 domains; the term "F(ab')² fragment" refers to an antibody fragment comprising two Fab fragments linked by a disulfide bridge at the hinge region; the term "Fab' fragment" refers to a fragment obtained by reducing the disulfide bond linking the two heavy chain fragments in the F(ab')² fragment, which consists of a complete light chain and an Fd fragment of heavy chain (consisting of VH and CH1 domains).

As used herein, the term "Fv" refers to an antibody fragment consisting of VL and VH domains of a single arm of an antibody. The Fv fragment is generally considered to be the smallest antibody fragment that can form a complete antigen binding site. It is generally believed that the six CDRs confer antigen binding specificity to an antibody. However, even a single variable region (e.g., a Fd fragment, which contains only three CDRs specific for an antigen) can recognize and bind to an antigen, although its affinity may be lower than that of the complete binding site.

As used herein, the term "Fc" refers to an antibody fragment formed by disulfide bonding between the second and third constant regions of the first heavy chain of an antibody and the second and third constant regions of the second heavy chain. The Fc fragment of an antibody has a variety of different functions but does not participate in antigen binding.

As used herein, the term "LALA mutation" refers to the mutation of the 234th amino acid of the natural Fc fragment from L to A, and the 235th amino acid from L to A.

As used herein, the term "scFv" refers to a single polypeptide chain comprising VL and VH domains, wherein the VL and VH are connected by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, ed., Springer-Verlag, New York, pp. 269-315 (1994)). Such scFv molecule may have a general structure: NH2-VL-linker-VH-COOH or NH2-VH-linker-VL-COOH. A suitable prior art linker may consist of repeated GGGGS amino acid sequence or variant thereof. For example, a linker having the amino acid sequence (GGGGS)4 may be used, but variants thereof may also be used (Holliger et al. (1993), Proc. Natl. Acad. Sci. USA 90: 6444-6448). Other linkers that can be used in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol. In some cases, there may also be a disulfide bond between the VH and VL of the scFv. In certain embodiments of the present invention, the scFv may form a di-scFv, which refers to an antibody formed by connecting two or more single scFvs in series. In certain embodiments of the present invention, scFv can form (scFv)2, which refers to an antibody formed by two or more single scFvs in parallel.

As used herein, the term "single-domain antibody (sdAb)" has the meaning generally understood by those skilled in the art, which refers to an antibody fragment composed of a single monomer variable antibody domain (e.g., a single heavy chain variable region), which retains the ability to specifically bind to the same antigen bound by a full-length antibody. Single-domain antibody is also called nanobody.

Each of the above antibody fragments retains the ability to specifically bind to the same antigen bound by a full-length antibody, and/or competes with the full-length antibody for specific binding to the antigen.

Antigen-binding fragments of an antibody (e.g., the above antibody fragments) can be obtained from a given antibody (e.g., the antibody provided by the present invention) using conventional techniques known to those skilled in the art (e.g., recombinant DNA technology or enzymatic or chemical cleavage methods), and the antigen-binding fragments of the antibody can be screened for specificity in the same manner as for intact antibody.

As used herein, unless the context clearly indicates otherwise, when the term "antibody" is referred to, it includes not only intact antibody, but also antigen-binding fragments of the antibody.

As used herein, the term "chimeric antibody" refers to an antibody in which a portion of the light chain or/and heavy chain is derived from one antibody (which may be derived from a particular species or belong to a particular antibody class or subclass), and another portion of the light chain or/and heavy chain is derived from another antibody (which may be derived from the same or different species or belong to the same or different antibody class or subclass), but in any case, it still retains binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include an antibody in which the heavy chain and light chain variable regions of the antibody are derived from a first antibody, and the heavy chain and light chain constant regions of the antibody are derived from a second antibody.

As used herein, the term "identity" is used to refer to the matching of sequences between two polypeptides or between two nucleic acids. To determine the percent identity of two amino acid sequences or two nucleic acid sequences, the sequences are aligned for optimal comparison purposes (e.g., a gap may be introduced in a first amino acid sequence or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between the two sequences is a function of the number of identical positions shared by the sequences (i.e., percent identity = number of identical overlapping positions/total number of positions × 100%). In certain embodiments, the two sequences are of the same length.

The determination of the percent identity between two sequences can also be accomplished using a mathematical algorithm. A non-limiting example of a mathematical algorithm for the comparison of two sequences is the algorithm of Karlin and Altschul, 1990, Proc. Natl. Acad. Sci. U.S.A. 87:2264-2268, as modified by Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403.

As used herein, the term "variant", in the context of polypeptide (including a polypeptide), also refers to a polypeptide or peptide comprising an amino acid sequence that has been altered by the introduction of substitution, deletion or addition of amino acid residues. In some cases, the term "variant" also refers to a polypeptide or peptide that has been modified (i.e., by covalently attaching any type of molecule to the polypeptide or peptide). For example, but not limiting, the polypeptide can be modified, for example, by glycosylation, acetylation, pegylation, phosphorylation, amidation, derivatization by a known protecting/blocking group, proteolytic cleavage, attachment to cellular ligand or other protein, etc. Derivatized polypeptides or peptides can be produced by chemical modification using techniques known to those skilled in the art, including but not limited to specific chemical cleavage, acetylation, formylation, metabolic synthesis of tunicamycin, etc. In addition, the variant has similar, identical or improved functions to the polypeptide or peptide from which it is derived.

As used herein, the term "specific binding" refers to a non-random binding reaction between two molecules, such as a reaction between an antibody and an antigen to which it is directed. The strength or affinity of a specific binding interaction can be expressed by the equilibrium dissociation constant (KD) of the interaction. In the present invention, the term "KD" refers to the dissociation equilibrium constant of a specific antibody-antigen interaction, which is used to describe the binding affinity between an antibody and an antigen. The smaller the equilibrium dissociation constant, the tighter the antibody-antigen binding and the higher the affinity between the antibody and the antigen.

The specific binding properties between two molecules can be determined using methods known in the art. One method involves measuring the rate of formation and dissociation of antigen binding sites/antigen complexes. Both the "association rate constant" (ka or kon) and the "dissociation rate constant" (kdis or koff) can be calculated from the concentrations and the actual rates of association and dissociation (see, Malmqvist M, Nature, 1993, 361: 186-187). The ratio of kdis/kon is equal to the dissociation constant KD (see, Davies et al., Annual Rev Biochem, 1990; 59: 439-473). The values of KD, kon and kdis can be measured by any effective method. In certain embodiments, the dissociation constant can be measured in Biacore using surface plasmon resonance (SPR). In addition, the dissociation constant can be measured by bioluminescence interferometry or Kinexa.

As used herein, the detectable label of the present invention can be any substance that can be detected by fluorescence, spectroscopy, photochemistry, biochemistry, immunology, electrical, optical or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), luminescent substances (e.g., chemiluminescent substances, such as acridinium ester compounds, luminol and its derivatives, ruthenium derivatives such as terpyridine ruthenium), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels.

As used herein, the term "vector" refers to a nucleic acid vehicle into which a polynucleotide can be inserted. When a vector can express the protein encoded by the inserted polynucleotide, the vector is called an expression vector. The vector can be introduced into a host cell by transformation, transduction or transfection, so that the genetic material elements it carries are expressed in the host cell. Vectors are well known to those skilled in the art, and include but are not limited to: plasmids; phagemids; cosmids; artificial chromosomes, such as yeast artificial chromosome (YAC), bacterial artificial chromosome (BAC) or P1-derived artificial chromosome (PAC); bacteriophages such as λ phage or M13 phage and animal viruses. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including but not limited to promoter sequence, transcription start sequence, enhancer sequence, selection element and reporter gene. In addition, the vector may also contain a replication start site.

As used herein, the term "host cell" refers to a cell that can be used to introduce a vector, including but not limited to prokaryotic cells such as *Escherichia coli* or *Bacillus subtilis,* fungal cells such as yeast cells or *Aspergillus,* insect cells such as *S2 Drosophila* cells or Sf9, or animal cells such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutical composition" is a product comprising one or more active ingredients (e.g., antibodies, small molecule drugs) in optionally specific amounts, and any product directly or indirectly produced by combining one or more active ingredients in optionally specific amounts. Different active ingredients in the pharmaceutical composition can be administered independently in separate formulations, which may be administered simultaneously or at different time points for combined synergy. In the present disclosure, "pharmaceutical composition" and "formulation" are not mutually exclusive.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, but not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), a solution containing a surfactant (e.g., 0.01% polysorbate 20), a pH buffer solution (e.g., a phosphate buffer solution), Ringer's solution, and any combination thereof.

As used herein, the term "prevention" refers to a method implemented to prevent or delay the occurrence of a disease, disorder, or symptom in a subject. As used herein, the term "treatment" refers to a method implemented to obtain a beneficial or desired clinical result. For the purposes of the present invention, beneficial or desired clinical results include (but are not limited to) alleviation of symptoms, reduction of the extent of the disease, stabilization (i.e., no longer worsening) of the state of the disease, delay or slowing the progression of the disease, improvement or alleviation of the state of the disease, and relief of symptoms (whether partial or complete), whether detectable or undetectable. In addition, "treatment" can also refer to prolonging survival compared to the expected survival (if not receiving treatment).

As used herein, the term "subject" refers to a mammal, such as a human, a cynomolgus monkey, or a mouse. In certain embodiments, the subject (e.g., a human, a cynomolgus monkey, or a mouse) suffers from a disease associated with c-Met, or is at risk of suffering from the above-mentioned disease.

As used herein, the term "effective amount" refers to an amount sufficient to obtain or at least partially obtain the desired effect. For example, a disease-preventing effective amount refers to an amount sufficient to prevent, arrest, or delay the occurrence of the disease; a disease-treating effective amount refers to an amount sufficient to cure or at least partially prevent the disease and its complications in a patient already suffering from the disease. Determining such an effective amount is well within the capabilities of those skilled in the art. For example, the amount effective for therapeutic use will depend on the severity of the disease to be treated, the overall state of the patient's own immune system, the patient's general condition such as age, weight and gender, the mode of administration of drug, and other treatments administered at the same time, etc.

As used herein, the term "single-armed antibody" refers to an antigen-binding fragment comprising a Fab segment and an Fc segment, generally the Fab segment comprises a heavy chain (e.g., VH and CH1) and a light chain (e.g., VL and CL), and the Fc segment comprises a constant region (e.g., CH2 and CH3). The Fab segment and the Fc segment may be connected by a linker or not. The single-armed antibody of the present invention can be prepared or synthesized by a variety of methods, for example, constructing the sequence encoding the Fab heavy chain and the sequence encoding the Fc into the same vector, and constructing the sequence encoding the Fab light chain into another vector, and transforming the two vectors into a host cell separately to obtain a single-armed antibody.

As used herein, the term "bispecific antibody" refers to a conjugate formed by a first antibody (or a fragment thereof) and a second antibody (or a fragment thereof) or an antibody analog through a coupling arm, and the coupling method includes but is not limited to chemical reaction, gene fusion and enzymatic. Bispecific antibodies can be linked or generated by various methods, for example, see the method of Songsivilai et al. (Clin. Exp. Immunol., 79: 315-321 (1990)), and the method of Kostelny et al. (J. Immunol., 148: 1547-1553 (1992)).

As used herein, the term "fucosylation" or "modification by fucosylation" has the same meaning, and refers to the presence of fucose residue in the oligosaccharide attached to the peptide backbone of an antibody. Correspondingly, the term "afucosylation" or "defucosylation" or "modification by defucosylation" has the same meaning, and refers to the removal of fucose residue in the oligosaccharide attached to the peptide backbone of an antibody.

### Beneficial effects of the invention

In the present application, an antibody or antigen-binding fragment thereof capable of specifically binding to c-Met is obtained, and further, a bispecific antibody capable of specifically binding to c-Met and EGFR, and an afucosylated bispecific antibody are prepared. Compared with the benchmark antibody, the bispecific antibody or afucosylated bispecific antibody of the present application is capable of: blocking HGF-dependent TKI resistance; blocking HGF-induced tumor cell proliferation and migration; inducing ADCC effect; inhibiting tumor growth in vivo, which is superior to the control antibody and the commercially available antibody Rybrevant; and exhibiting synergistic tumor killing effect when combined with an EGFR inhibitor. Therefore, the bispecific antibody of the present application has great clinical value.

The embodiments of the present invention will be described in detail below in conjunction with the drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

### Brief Description of the Drawings

Fig. 1 shows a schematic diagram of the structure of single-arm antibody for anti-c-Met screening (Fig. 1A) and the anti-EGFRxc-Met bispecific antibody (Fig. 1B) of the present application.
Fig. 2 shows the inhibitory effects of the anti-c-Met antibodies (136 single-arm antibody, 187 single-arm antibody) of the present application and the control antibody on the growth of HCC827 cells.
Fig. 3 shows the results of blocking EGF-EGFR interaction by the anti-EGFRxc-MET bispecific antibodies (EGFRxc-Met-136, EGFRxc-Met-187) of the present application and the control antibody.
Fig. 4 shows the inhibitory effects of the anti-EGFRxc-Met bispecific antibodies (EGFRxc-Met-136, EGFRxc-Met-187) of the present application and the control antibody on the growth of HCC827 cells.
Fig. 5 shows the inhibitory effects of the anti-EGFRxc-Met bispecific antibodies (EGFRxc-Met-136, EGFRxc-Met-187) of the present application and the control antibody on the proliferation of H596 cells.
Fig. 6 shows the blocking effects of the anti-EGFRxc-Met bispecific antibodies (EGFRxc-Met-136, EGFRxc-Met-187) of the present application and the control antibody on the migration of HGF-induced HepG2 cells.
Fig. 7 shows the quality identification results of the stable cell line products, wherein Fig. 7A shows the purity of the bispecific antibody obtained by affinity purification; and Fig. 7B shows the ratio of the correctly paired product of purified bispecific antibody.

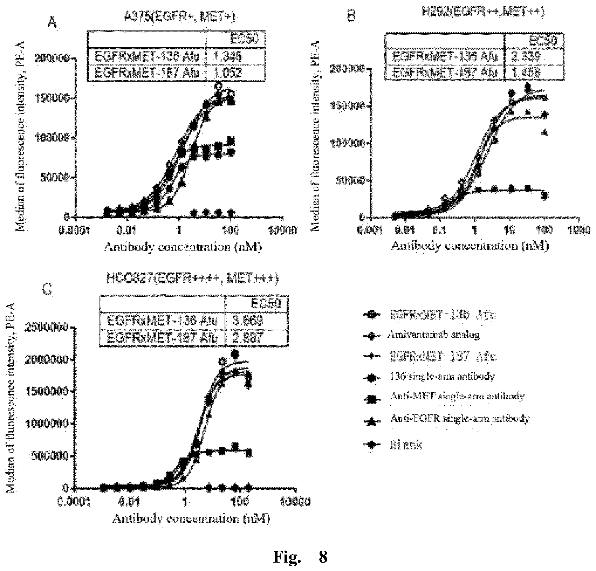

Fig. 8 shows the cell binding activity results of the afucosylated bispecific antibodies (EGFRxc-Met-136 Afu) and (EGFRxc-Met-187 Afu) as well as the control antibody to A375 (Fig. 8A), H292 (Fig. 8B), and HCC827 (Fig. 8C) cells.
Fig. 9 shows the ADCC effects induced by the anti-EGFRxc-Met bispecific antibodies (EGFRxc-Met-136, EGFRxc-Met-187), the afucosylated bispecific antibody (EGFRxc-Met-136 Afu) of the present application and the control antibody to A375 (Fig. 9A), H1975 (Fig. 9B), and HCC827 (Fig. 9C) cells.
Fig. 10 shows the killing effects of PBMC on A375 cells induced by the anti-EGFRxc-Met bispecific antibodies (EGFRxc-Met-136, EGFRxc-Met-187), afucosylated bispecific antibody (EGFRxc-Met-136 Afu) of the present application and the control antibody.
Fig. 11 shows the killing effects of PBMC on HCC827 cells induced by the combination of afucosylated anti-EGFRxc-Met bispecific antibody (EGFRxc-Met-136 Afu) of the present application and the EGFR inhibitor.
Fig. 12 shows the inhibitory effects of the afucosylated anti-EGFRxc-Met bispecific antibody (EGFRxc-Met-136 Afu, EGFRxc-Met-187 Afu) of the present application and the control antibody on the growth of H292 human lung cancer cells.
Fig. 13 shows the inhibitory effects of different doses of the afucosylated anti-EGFRxc-Met bispecific antibody (EGFRxc-Met-136 Afu) of the present application on the growth of H1975 human lung cancer cells.
Fig. 14 shows the inhibitory effects of different doses of the afucosylated anti-EGFRxc-Met bispecific antibody (EGFRxc-Met-136 Afu) of the present application on the growth of H292 human lung cancer cells.
Fig. 15 shows the inhibitory effects of a single dose of the afucosylated anti-EGFRxc-Met bispecific antibody (EGFRxc-Met-136 Afu) of the present application and the control commercial antibody Rybrevant on the growth of H292 human lung cancer cells.
Fig. 16 shows the inhibitory effects of the combination of the afucosylated anti-EGFRxc-Met bispecific antibody (EGFRxc-Met-136 Afu) of the present application and the small molecule inhibitor (osimertinib) on the growth of H1975-HGF human lung cancer cells.

### Sequence information

The information of some sequences involved in the present invention is provided in Table 1 below.

**Table 1: Description of sequences**

| SEQ ID NO: | Description | Sequence |
|---|---|---|
| 1 | EGFR VH | |
| 2 | EGFR-1 Fc | QVQLVESGGGVVQPGRSLRLSCAASGFTFSTYGMHWVRQAPGKGLEWVAVIW DDGSYKYYGDSVKGRFTISRDNSKNTLYLQMNSLRAEDTAVYYCARDGITMV RGVMKDYFDYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQ DWLNGKEYKCKVSNKALPAPIEKTISKAKGQPREPQVYTEPPSRDELTKNQVSL TCLVKGFYPSDIAVEWESNGQPENNYKTTPPVLDSDGSFFLLSVLTVDKSRWQQ GNVFSCSVMHEALHNHYTQKSLSLSPGK |
| 3 | EGFR VL | |
| 4 | EGFR LC | AIQLTQSPSSLSASVGDRVTITCRASQDISSALVWYQQKPGKAPKLLIYDASSLES GVPSRFSGSESGTDFTLTISSLQPEDFATYYCQQFNSYPLTFGGGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| 5 | cMet VH | QVQLVQSGAEVKKPGASVKVSCETSGYTFTSYGISWVRQAPGHGLEWMGWIS AYNGYTNYAQKLQGRVTMTTDTSTSTAYMELRSLRSDDTAVYYCARDLRGTN YFDYWGQGTLVTVSS |
| 6 | cMet-2 Fc | QVQLVQSGAEVKKPGASVKVSCETSGYTFTSYGISWVRQAPGHGLEWMGWIS AYNGYTNYAQKLQGRVTMTTDTSTSTAYMELRSLRSDDTAVYYCARDLRGTN YFDYWGQGTLVTVSSASTKGPSVFPLAPSSKSTSGGTAALGCLVKDYFPEPVTV |
| | | SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLLCLVK GFYPSDIAVEWESNGQPENNYKTTPPVLRSDGSFFLYSKLTVDKSRWQQGNVFS CSVMHEALHNHYTQKSLSLSPGK |
| 7 | cMet VL | DIQMTQSPSSVSASVGDRVTITCRASQGISNWLAWFQHKPGKAPKLLIYAASSL LSGVPSRFSGSGSGTDFTLTISSLQPEDFATYYCQQANSFPITFGQGTRLEIK |
| 8 | cMet LC | DIQMTQSPSSVSASVGDRVTITCRASQGISNWLAWFQHKPGKAPKLLIYAASSL LSGVPSRFSGSGSGTDFTLTISSLQPEDFATYYCQQANSFPITFGQGTRLEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 9 | 136 VH | EVQLQESGPGLVKPSETLSLTCTVSGGSVTSVNYYWKWIRQPPGKGLEWIGYIS YSGNTNYNPSLKSRVTISVDTSKNQFSLKLSSVTAADTAVYYCVRAPYYYMDV WSKGTTVTVSS |
| 10 | 136 -2 Fc | EVQLQESGPGLVKPSETLSLTCTVSGGSVTSVNYYWKWIRQPPGKGLEWIGYIS YSGNTNYNPSLKSRVTISVDTSKNQFSLKLSSVTAADTAVYYCVRAPYYYMDV WSKGTTVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLLCLVKGFYPS DIAVEWESNGQPENNYKTTPPVLRSDGSFFLYSKLTVDKSRWQQGNVFSCSVM HEALHNHYTQKSLSLSPG |
| 11 | 136 VL | DIVMTQSPSSVSASVGDRVTITCRASQGISSWLAWYQQKPGKAPKLLIYAVSSL QSGVPSRFSGSGSGTDFTLTISSLQPEDFATYFCQQANSFPLTFGGGTKVEIK |
| 12 | 136 LC | DIVMTQSPSSVSASVGDRVTITCRASQGISSWLAWYQQKPGKAPKLLIYAVSSL QSGVPSRFSGSGSGTDFTLTISSLQPEDFATYFCQQANSFPLTFGGGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 13 | 187 VH | ELQLQESGPGLVKPSETLSLTCTVSGGSISSSSYYWGWIRQSPGKGLEWIGSRYY SGNTYYNPSLKSRVTMSVDTSKNQFSLKLRSVTAADTAVYYCARQVYDYWRD WGQGALVTVSS |
| 14 | 187 -2Fc | ELQLQESGPGLVKPSETLSLTCTVSGGSISSSSYYWGWIRQSPGKGLEWIGSRYY SGNTYYNPSLKSRVTMSVDTSKNQFSLKLRSVTAADTAVYYCARQVYDYWRD WGQGALVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLLCLVKGFYPS DIAVEWESNGQPENNYKTTPPVLRSDGSFFLYSKLTVDKSRWQQGNVFSCSVM HEALHNHYTQKSLSLSPG |
| 15 | 187 VL | DIVMTQSPSSVSASVGDRVTITCRASQGISSWLAWYQQKPGKAPNLLIYAASNL PSGVPSRFSGSGSGTVFTLTISSLQPEDFATYYCQQSNSFPLTFGGGTKVEIK |
| 16 | 187 LC | DIVMTQSPSSVSASVGDRVTITCRASQGISSWLAWYQQKPGKAPNLLIYAASNL PSGVPSRFSGSGSGTVFTLTISSLQPEDFATYYCQQSNSFPLTFGGGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 17 | IgG1-CH1-Fc (LALA mutation, knob mutation) | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHT |
| | | CPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVYTLPPCRDELTKNQVSLWCLVKGFYPSDIAVEWESNG QPENNYKTTPPVLDSDGSFFLYSKLTVDKSRWQQGNVFSCSVMHEALHNHYTQ KSLSLSPG |
| 18 | Fc-LALA-hole | DKTHTCPPCPAPEAAGGPSVFLFPPKPKDTLMISRTPEVTCVVVDVSHEDPEVKF NWYVDGVEVHNAKTKPREEQYNSTYRVVSVLTVLHQDWLNGKEYKCKVSNK ALPAPIEKTISKAKGQPREPQVCTLPPSRDELTKNQVSLSCAVKGFYPSDIAVEW ESNGQPENNYKTTPPVLDSDGSFFLVSKLTVDKSRWQQGNVFSCSVMHEALHN HYTQKSLSLSPG |
| 19 | SET1 CH | ASTKGPSVFPLAPSSKSTSGGTAALGCQVEDYFPEPVTVSWNSGALTSGVHTFP AVLQSSGLYELSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVYTLPPCRDELTKNQVSLWCLVKGFYPSDIAVEWESNG QPENNYKTTPPVLDSDGSFFLYSKLTVDKSRWQQGNVFSCSVMHEALHNHYTQ KSLSLSPG |
| 20 | SET2 CH | ASTKGPSVFPRAPSSKSTSGGTAALGCLVRDYFPEPVTVSWNSGALTSGVHTFP AVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVCTLPPSRDELTKNQVSLSCAVKGFYPSDIAVEWESNGQ PENNYKTTPPVLDSDGSFFLVSKLTVDKSRWQQGNVFSCSVMHEALHNHYTQK SLSLSPG |
| 21 | SET1 CL | RTVAAPSVFIFPPSDEQLKSGRASVVCLLNNFYPREAKVQWKVDNALQSGNSQ ESVTEQDSKDSTYSLSSRLQLSKADYEKHKVYACEVTHQGLSSPVTKSFNRGEC |
| 22 | SET2 CL | RTVAAPSVFIFPPSDEELKSGTASVQCLLNNFYPREAKVQWKVDNALQSGNSQE SVTEQDSKDSTYSLSSELTLSKADYEKHKVYACEVTHQGLSSPVTKSFNRGEC |
| 23 | final cMet HC | EVQLQESGPGLVKPSETLSLTCTVSGGSVTSVNYYWKWIRQPPGKGLEWIGYIS YSGNTNYNPSLKSRVTISVDTSKNQFSLKLSSVTAADTAVYYCVRAPYYYMDV WSKGTTVTVSSASTKGPSVFPLAPSSKSTSGGTAALGCQVEDYFPEPVTVSWNS GALTSGVHTFPAVLQSSGLYELSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPCRDELTKNQVSLWCLVKGFYP SDIAVEWESNGQPENNYKTTPPVLDSDGSFFLYSKLTVDKSRWQQGNVFSCSV MHEALHNHYTQKSLSLSPG |
| 24 | final cMet LC | DIVMTQSPSSVSASVGDRVTITCRASQGISSWLAWYQQKPGKAPKLLIYAVSSL QSGVPSRFSGSGSGTDFTLTISSLQPEDFATYFCQQANSFPLTFGGGTKVEIKRTV AAPSVFIFPPSDEQLKSGRASVVCLLNNFYPREAKVQWKVDNALQSGNSQESVT EQDSKDSTYSLSSRLQLSKADYEKHKVYACEVTHQGLSSPVTKSFNRGEC |
| 25 | final EGFR HC | EVQLVESGGGVVQPGRSLRLSCAASGFTFSTYGMHWVRQAPGKGLEWVAVIW DDGSYKYYGDSVKGRFTISRDNSKNTLYLQMNSLRAEDTAVYYCARDGITMV RGVMKDYFDYWGQGTLVTVSSASTKGPSVFPRAPSSKSTSGGTAALGCLVRDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQ DWLNGKEYKCKVSNKALPAPIEKTISKAKGQPREPQVCTLPPSRDELTKNQVSL SCAVKGFYPSDIAVEWESNGQPENNYKTTPPVLDSDGSFFLVSKLTVDKSRWQ QGNVFSCSVMHEALHNHYTQKSLSLSPG |
| 26 | final EGFR LC | DIQLTQSPSSLSASVGDRVTITCRASQDISSALVWYQQKPGKAPKLLIYDASSLES GVPSRFSGSESGTDFTLTISSLQPEDFATYYCQQFNSYPLTFGGGTKVEIKRTVA |
| | | APSVFIFPPSDEELKSGTASVQCLLNNFYPREAKVQWKVDNALQSGNSQESVTE QDSKDSTYSLSSELTLSKADYEKHKVYACEVTHQGLSSPVTKSFNRGEC |
| 27 | 136-VH CDR1 | GGSVTSVNYY |
| 28 | 136-VH CDR2 | ISYSGNT |
| 29 | 136-VH CDR3 | VRAPYYYMDV |
| 30 | 136-VL CDR1 | QGISSW |
| 31 | 136-VL CDR2 | AVS |
| 32 | 136-VL CDR3 | QQANSFPLT |
| 33 | 187-VH CDR1 | GGSISSSSYY |
| 34 | 187-VH CDR2 | RYYSGNT |
| 35 | 187-VH CDR3 | ARQVYDYWRD |
| 36 | 187-VL CDR1 | QGISSW |
| 37 | 187-VL CDR2 | AAS |
| 38 | 187-VL CDR3 | QQSNSFPLT |
| 39 | Heavy chain constant region 1 | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVYTEPPSRDELTKNQVSLTCLVKGFYPSDIAVEWESNGQ PENNYKTTPPVLDSDGSFFLLSVLTVDKSRWQQGNVFSCSVMHEALHNHYTQK SLSLSPGK |
| 40 | Heavy chain constant region 2 | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVYTLPPSRDELTKNQVSLLCLVKGFYPSDIAVEWESNGQ PENNYKTTPPVLRSDGSFFLYSKLTVDKSRWQQGNVFSCSVMHEALHNHYTQK SLSLSPG |
| 41 | Light chain constant region | |
| 42 | EGFR-VH-Knob | QVQLVESGGGVVQPGRSLRLSCAASGFTFSTYGMHWVRQAPGKGLEWVAVIW |
| | | DDGSYKYYGDSVKGRFTISRDNSKNTLYLQMNSLRAEDTAVYYCARDGITMV RGVMKDYFDYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPEAAGGPSVFLFPPKPKDTLMISRTPEV TCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPCRDELTKNQVSL WCLVKGFYPSDIAVEWESNGQPENNYKTTPPVLDSDGSFFLYSKLTVDKSRWQ QGNVFSCSVMHEALHNHYTQKSLSLSPG |
| 43 | MET-VH-Knob | QVQLVQSGAEVKKPGASVKVSCETSGYTFTSYGISWVRQAPGHGLEWMGWIS AYNGYTNYAQKLQGRVTMTTDTSTSTAYMELRSLRSDDTAVYYCARDLRGTN YFDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPCRDELTKNQVSLWCLV KGFYPSDIAVEWESNGQPENNYKTTPPVLDSDGSFFLYSKLTVDKSRWQQGNV FSCSVMHEALHNHYTQKSLSLSPG |
| 44 | 136-VH-K nob | EVQLQESGPGLVKPSETLSLTCTVSGGSVTSVNYYWKWIRQPPGKGLEWIGYIS YSGNTNYNPSLKSRVTISVDTSKNQFSLKLSSVTAADTAVYYCVRAPYYYMDV WSKGTTVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPCRDELTKNQVSLWCLVKGFYP SDIAVEWESNGQPENNYKTTPPVLDSDGSFFLYSKLTVDKSRWQQGNVFSCSV MHEALHNHYTQKSLSLSPG |
| 45 | 187-VH-K nob | ELQLQESGPGLVKPSETLSLTCTVSGGSISSSSYYWGWIRQSPGKGLEWIGSRYY SGNTYYNPSLKSRVTMSVDTSKNQFSLKLRSVTAADTAVYYCARQVYDYWRD WGQGALVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPCRDELTKNQVSLWCLVKGFYP SDIAVEWESNGQPENNYKTTPPVLDSDGSFFLYSKLTVDKSRWQQGNVFSCSV MHEALHNHYTQKSLSLSPG |
| 46 | EGFR-VH CDR1 | GFTFSTYG |
| 47 | EGFR-VH CDR2 | IWDDGSYK |
| 48 | EGFR-VH CDR3 | ARDGITMVRGVMKDYFDY |
| 49 | EGFR-VL CDR1 | QDISSA |
| 50 | EGFR-VL CDR2 | DAS |
| 51 | EGFR-VL CDR3 | QQFNSYPLT |
| 52 | final EGFR VH | EVQLVESGGGVVQPGRSLRLSCAASGFTFSTYGMHWVRQAPGKGLEWVAVIW DDGSYKYYGDSVKGRFTISRDNSKNTLYLQMNSLRAEDTAVYYCARDGITMV RGVMKDYFDYWGQGTLVTVSS |
| 53 | final EGFR VL | DIQLTQSPSSLSASVGDRVTITCRASQDISSALVWYQQKPGKAPKLLIYDASSLES GVPSRFSGSESGTDFTLTISSLQPEDFATYYCQQFNSYPLTFGGGTKVEIK |

### Specific Models for Carrying Out the Invention

The present invention is now described with reference to the following examples which are intended to illustrate the present invention (but not to limit the present invention).

Unless otherwise specified, the experiments and methods described in the examples are basically carried out according to conventional methods well known in the art and described in various references. For example, conventional techniques of immunology, biochemistry, chemistry, molecular biology, microbiology, cell biology, genomics and recombinant DNA used in the present invention can be found in Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel et al., eds., (1987)); METHODS IN ENZYMOLOGY series (Academic Publishing Company): PCR 2: A PRACTICAL METHOD. APPROACH) (M.J. MacPherson, B.D. Hames and G.R. Taylor, ed. (1995)), and ANIMAL CELL CULTURE (R.I. Freshney, ed. (1987)).

In addition, if the specific conditions were not specified in the examples, they were carried out according to conventional conditions or conditions recommended by the manufacturer. The reagents or instruments used without indicating the manufacturer were all conventional products that could be obtained commercially. It is known to those skilled in the art that the examples describe the present invention by way of example and are not intended to limit the scope of protection of the present invention. All the disclosures and other references mentioned herein are incorporated herein by reference in their entirety.

### Example 1: Antibody construction and protein expression and purification

### Screening of monoclonal antibodies

The sequences of anti-c-MET antibodies 136 and 187 used in the present application were obtained by immunizing mice with cellular mesenchymal epithelial transition factor (c-Met) antigen (purchased from AcroBiosystems), extracting total RNA and performing reverse transcription, and screening yeast display libraries constructed by PCR amplification. The sequences of antibodies 136 and 187 were obtained by sequencing, wherein the CDR sequences of the antibodies were determined by the IMGT numbering system (Lefranc et al., Dev. Comparat. Immunol. 27:55-77, 2003). Specifically, a fully humanized anti-c-MET antibody library was first constructed from five immunized mice, which had a diversity of 3×10^7, and human c-MET protein was labeled according to the product instructions of the biotin labeling kit (purchased from Thermo). Yeast capable of specifically binding to c-MET was enriched by MACS, and yeast cells that were capable of specifically binding to biotin-labeled c-MET protein had high affinity were finally obtained after multiple rounds of flow cytometry sorting. The sequences of the heavy and light chains of the antibody were retrieved using the template of the yeast that was finally selected, and these sequences were then constructed into an expression vector to prepare antibodies.

After the antibodies were prepared, the ForteBio affinity assay was performed according to the reported method (Estep, P et al., Determination of antibody-antigen affinity and epitope binding based on high-throughput methods. MAbs, 2013.5(2):p.270-8). The results showed that anti-c-MET antibodies 136 and 187 had good binding activity with human c-MET protein and monkey c-MET protein (Table 2, Table 3).

**Table 2. Affinity of candidate molecules for human c-Met**

| No. | KD(M) | Kon(1/Ms) | Koff(1/s) |
|---|---|---|---|
| 136 | 2.93E-11 | 2.29E+05 | 6.71E-06 |
| 187 | 1.68E-09 | 2.20E+05 | 3.69E-04 |

**Table 3. Affinity of candidate molecules for monkey c-Met**

| No. | KD(M) | Kon(1/Ms) | Koff(1/s) |
|---|---|---|---|
| 136 | 1.16E-09 | 3.69E+05 | 4.26E-04 |
| 187 | 2.92E-09 | 3.24E+05 | 9.47E-04 |

Construction of bispecific antibodies targeting cMet and EGFR was performed according to the method described in the patent application (patent application number: 201611016435.0), nucleotide sequences encoding the variable regions of anti-EGFR and anti-cMet antibodies were synthesized, and ligated to the constant region (the sequence was from the patent application: PCT/CN2017/111310) that could spontaneously form heterodimers. Among them, 3 different anti-cMet antibodies were selected. The first anti-cMet antibody was the antibody disclosed in patent application: WO2011/110642A2 (its sequence was shown in Table 1), which was used as a control antibody; the second and third anti-cMet antibodies were antibodies 136 and 187 obtained from the above screening, and their sequences were shown in Table 1.

The nucleotide sequence encoding the anti-EGFR antibody heavy chain variable region (its amino acid sequence was set forth in SEQ ID NO: 1, which was disclosed in the patent application: WO02/100348A2) was conventionally synthesized, ligated to the nucleotide sequence encoding the heavy chain constant region sequence 1 (SEQ ID NO: 39) that could spontaneously form a heterodimer, then added with EcoRI and XhoI restriction enzyme sites at both ends, and cloned into the vector pCDNA3.1 (Genwiss) to construct the plasmid EGFR-HC-pCDNA3.1 (the amino acid sequence of EGFR-1 Fc was set forth in SEQ ID NO: 2); the nucleotide sequence encoding the anti-EGFR antibody light chain variable region (its amino acid sequence was set forth in SEQ ID NO: 3, which was disclosed in the patent application: WO02/100348A2) was conventionally synthesized, ligated to the nucleotide sequence encoding the light chain constant region sequence (SEQ ID NO: 41), then added with EcoRI and XhoI restriction enzyme sites at both ends, and cloned into the vector pCDNA3.1 to construct the plasmid EGFR-LC-pCDNA3.1 (the amino acid sequence of EGFR-LC was set forth in SEQ ID NO: 4).

The nucleotide sequence encoding the anti-cMet control antibody heavy chain variable region (SEQ ID NO: 5, patent application: WO2011/110642A2) was conventionally synthesized, ligated to the nucleotide sequence encoding the heavy chain constant region sequence 2 (SEQ ID NO: 40) that could spontaneously form a heterodimer, then added with EcoRI and XhoI restriction enzyme sites at both ends, and cloned into the vector pCDNA3.1 to construct a plasmid cMetGen-HC-pCDNA3.1 (the amino acid sequence of cMet-2 Fc was set forth in SEQ ID NO: 6); the nucleotide sequence encoding the anti-cMet control antibody light chain variable region (SEQ ID NO: 7, patent application: WO2011/110642A2) was conventionally synthesized, ligated to the nucleotide sequence encoding the light chain constant region sequence (SEQ ID NO: 41), then added with EcoRI and XhoI restriction enzyme sites at both ends, and cloned into the vector pCDNA3.1 to construct a plasmid cMetGen-LC-pCDNA3.1 (the amino acid sequence of cMet LC was set forth in SEQ ID NO: 8).

The nucleotide sequence encoding the anti-cMet antibody heavy chain variable region (SEQ ID NO: 9) was conventionally synthesized, ligated to the nucleotide sequence encoding the heavy chain constant region sequence 2 (SEQ ID NO: 40) that could spontaneously form a heterodimer, then added with EcoRI and XhoI restriction enzyme sites at both ends, and cloned into the vector pCDNA3.1 to construct the plasmid cMet136-HC-pCDNA3.1 (the amino acid sequence of 136-2 Fc was set forth in SEQ ID NO: 10); the nucleotide sequence encoding the anti-cMet antibody light chain variable region (SEQ ID NO: 11) was conventionally synthesized, ligated to the nucleotide sequence encoding the light chain constant region sequence (SEQ ID NO: 41), then added with EcoRI and XhoI restriction enzyme sites at both ends, and cloned into the vector pCDNA3.1 to construct the plasmid cMet136-LC-pCDNA3.1 (the amino acid sequence of 136 LC was set forth in SEQ ID NO: 12).

The nucleotide sequence encoding the anti-cMet antibody heavy chain variable region (SEQ ID NO: 13) was conventionally synthesized, ligated to the nucleotide sequence encoding the heavy chain constant region sequence 2 (SEQ ID NO: 40) that could spontaneously form a heterodimer, then added with EcoRI and XhoI restriction enzyme sites at both ends, and cloned into the vector pCDNA3.1 to construct the plasmid cMet187-HC-pCDNA3.1 (the amino acid sequence of 187-2Fc was set forth in SEQ ID NO: 14); the nucleotide sequence encoding the anti-cMet antibody light chain variable region (SEQ ID NO: 15) was conventionally synthesized, ligated to the nucleotide sequence encoding the light chain constant region sequence (SEQ ID NO: 41), then added with EcoRI and XhoI restriction enzyme sites at both ends, and cloned into the vector pCDNA3.1 to construct the plasmid cMet187-LC-pCDNA3.1 (the amino acid sequence of 187 LC was set forth in SEQ ID NO: 16).

The homologous recombination product was transferred into Top10 competent cells, the cells were coated on an ampicillin resistant plate, and cultured overnight at 37°C, and single colonies were picked for sequencing.

### Cell transfection and protein expression

The normal host cell line (ExpiCHO-S cell F1) (Yida) and the afucosylation cell line with gene knockout (CHOS-ADP Fut8 KO) (self-constructed by BIOTHEUS; Fut8 knockout CHOS cells were obtained by knocking out the FUT8 allele of CHO cells) were used for electroporation transient expression scheme for transfection and expression of normal antibodies and afucosylated antibodies. The specific method was referred to the product manual. The supernatant was collected after 13 days of cell culture.

### Protein purification

The target protein was purified using a Protein A affinity chromatography column (MabSelect^{™} PrismA, GE Healthcare). The purification column was equilibrated with 5 to 10 column volumes of equilibrium buffer (20 mM Tris-HCl, 150 mM NaCl, pH7.4) until the conductivity and pH of the effluent remained unchanged, and then loading was performed. After loading, the column was rinsed continuously with the equilibration buffer until the UV value of the effluent no longer decreased. Elution buffer (20 mM glycine-HCl, pH2.7) was used to elute the sample, and the effluent was collected. The eluent was neutralized with alkaline buffer (1 M Tris-HCl, pH8.0).

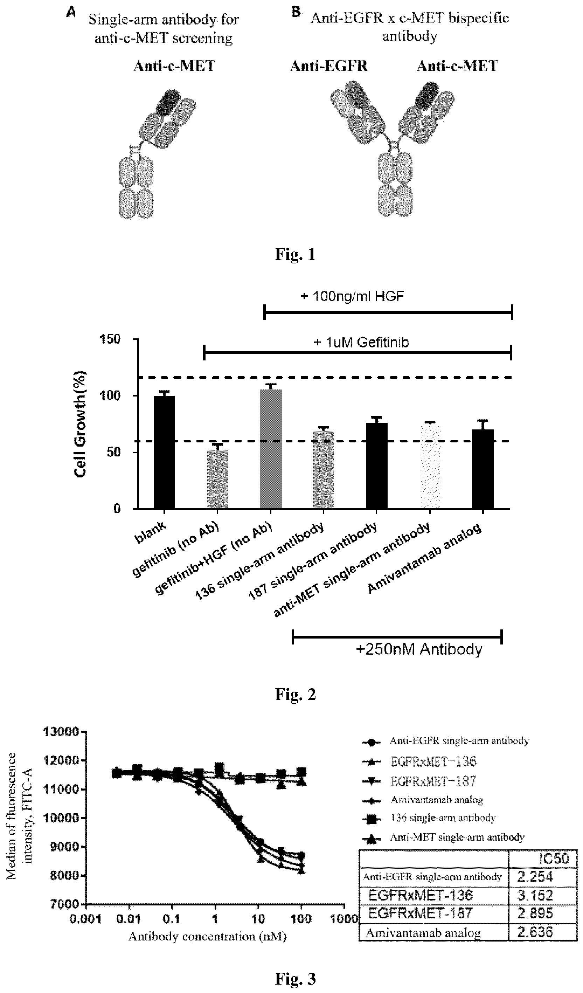

After the monoclonal antibody was obtained, it was concentrated and exchanged into PBS buffer to reach a final protein concentration of 5 to 10 mg/mL. The anti-cMet antibody and the anti-EGFR antibody were mixed in a molar ratio of 1:1, added with the reducing agent DTT to perform the reduction at 4°C for 4 hours. Then the solution was changed to the buffer of 20 mM sodium phosphate, pH 6.0, and DTT was removed. Fragments and aggregates were removed by a using a cation exchange method. The components with higher purity were collected and combined, and the protein was allowed to fully oxidize in the air. The antibody Fc segment would form heterodimer, thereby obtaining 5 anti-EGFRxcMet bispecific antibody proteins, and their structures were shown in Fig. 1B. The antibodies obtained in normal host cells were named Amivantamab analog (control antibody), EGFRxMET-136 and EGFRxMET-187. The antibodies obtained from the afucosylation cell line with gene knockout were named EGFRxMET-187 Afu and Amivantamab analog Afu.

### Construction and preparation of single-arm control antibodies

The nucleotide sequence encoding the anti-EGFR antibody heavy chain variable region (SEQ ID NO: 1) and the nucleotide sequences of three anti-cMet antibody heavy chain variable regions (SEQ ID NOs: 5, 9, 13) were linked to the nucleotide sequence encoding the human IgG1-CH1-Fc (LALA mutation, knob mutation) segment (SEQ ID NO: 17) to obtain the nucleotide sequence of the anti-EGFR antibody heavy chain (SEQ ID NO: 42) and three anti-cMet antibody heavy chains (SEQ ID NOs: 43, 44, 45), which were constructed into the EcoR I/Not I double-digestion linearized pCDNA3.1 vector using homologous recombinase (purchased from Vazyme). The nucleotide sequences encoding the four antibody light chains (SEQ ID NOs: 4, 8, 12, 16) were constructed into the EcoR I/Xhol I double-digestion linearized pCDNA3.1 vector, and the process was in accordance with the product instructions. The homologous recombination product was transferred into Top10 competent cells, coated on an ampicillin-resistant plate, and cultured at 37°C overnight, single clones were picked for sequencing, and plasmids were extracted. The nucleotide sequence encoding Fc-LALA-hole (SEQ ID NO: 18) was constructed into the EcoR I/Xhol I double-digestion linearized pCDNA3.1 vector.

By using ExpiCHO^{™} Expression System Kit (purchased from Thermo), the extracted three plasmids of heavy chain (Fc-LALA-knob), light chain and Fc-LALA-hole were co-transfected into Expi-CHO cells to form a single Fab antibody structure (Fig. 1A). The transfection method was in accordance with the product instructions. After 5 days of cell culture, the supernatant was collected and the target protein was purified by protein A magnetic beads (purchased from GenScript) sorting method. The magnetic beads were resuspended in an appropriate volume of binding buffer (PBS + 0.1% Tween 20, pH 7.4) (1 to 4 times the volume of magnetic beads) and added to the sample to be purified, and incubation was carried out at room temperature for 1 hour with gentle shaking during the period. The sample was placed on a magnetic stand (purchased from Beaver), the supernatant was discarded, and the magnetic beads were washed 3 times with binding buffer. Elution buffer (0.1M sodium citrate, pH3.2) was added according to 3 to 5 times the volume of the magnetic beads, and the mixture was shaken at room temperature for 5 to 10 minutes. The mixture was placed back on the magnetic stand, and the elution buffer was collected and transferred to a collection tube with neutralization buffer (1M Tris, pH 8.54) and mixed well. Four target proteins were obtained, which were named anti-EGFR single-arm antibody, anti-MET single-arm antibody, 136 single-arm antibody, and 187 single-arm antibody.

### Blocking of HGF-c-MET signaling pathway by c-MET antibodies 136 and 187

HCC827 cells were human non-small cell lung cancer cells (purchased from the Cell Bank of the Chinese Academy of Sciences), which highly express epidermal growth factor receptor EGFR (exon 19 deletion) and c-Met receptor. The treatment with small molecule tyrosine kinase inhibitor (TKI) Gefitinib (gefitinib, an epidermal growth factor receptor tyrosine kinase inhibitor) would induce apoptosis in HCC827 cells. If HGF was added at the same time under such conditions, the c-Met pathway would be activated to induce HCC827 to produce resistance to Gefitinib, thereby inhibiting apoptosis.

The cell density of the HCC827 cells after the expanded culture was adjusted to 2×10⁴ cells/ml, added to a 96-well cell culture plate at 100 µl/well, and cultured overnight for later use. The antibody to be tested was diluted to 1000 nM with 1640 medium, HGF was diluted to 800 ng/mL, and Gefitinib was diluted to 8 µM. According to the experimental requirements, the diluted antibody at 50 µl/well, HGF at 25 µl/well, and Gefitinib at 25 µl/well were added to the 96-well plate with HCC827 cells, and supplemented with 1640 medium to reach a total volume of 200 µl/well. After culturing for 3 days at 37°C and 5% carbon dioxide, 100 µl of the medium was removed, and then Cell titer glo (purchased from Promega) was added at 100 µl/well, and the chemiluminescent signal was collected with an microplate reader.

The experimental results were shown in Fig. 2. Anti-c-MET antibodies 136 and 187 could restore the proliferation inhibitory effect of Gefitinib on HCC827 cells by blocking the HGF-c-MET signaling pathway.

### Example 2: Anti-EGFRxc-Met bispecific antibody blocking EGF-EGFR interaction

H292 cells, which were human non-small cell lung cancer cells purchased from Procell, were capable of expressing EGFR and c-Met receptors. H292 cells were expanded to a suitable density, digested and detached from the cell culture flask, resuspended to 1×10⁶ cells/ml, added to a 96-well flow plate, 100 µL per well, and centrifuged for later use. The antibody to be tested was subjected to 3-fold dilution with PBS, starting from 200 nM, and the diluted sample was added to the 96-well flow plate with cells, 100 µL/well, and incubated at 25°C for 60 min. Biotin-h.EGF was diluted to 100 nM with PBS, added to the 96-well flow plate with cells, 100 µL/well, and incubated at 25°C for 60 min. then washed twice with PBS. Streptavidin-FITC (purchased from Jackson) was diluted 100 times with PBS and added to the plate, 100 µL/well, incubated at 4°C for 30 min, and washed twice with PBS. 100 µL/well of PBS was added to resuspend the cells, then the cells were detected on a CytoFlex (Beckman) flow cytometer, and the corresponding MFI was calculated.

As shown in Fig. 3, the candidate bispecific antibody molecules EGFRxMET-136 and EGFRxMET-187 of the present application both blocked the interaction between EGF and EGFR which expressed on H292 cells, with IC50 values of 3.15nM and 2.90nM, respectively. As negative controls, the anti-c-Met 136 single-arm antibody and the anti-c-Met single-arm molecule constructed based on the control molecule Amivantamab could not block the interaction between EGF and EGFR.

### Example 3: Anti-EGFRxc-Met bispecific antibody blocking HGF-dependent TKI resistance

In this experiment, the strength of the anti-EGFRxc-Met bispecific antibody of the present application in blocking HGF signal was detected. The expanded HCC827 cells (HCC827 cells were human non-small cell lung cancer cells purchased from the Cell Bank of the Chinese Academy of Sciences, which highly expressed epidermal growth factor receptor EGFR (exon 19 deletion) and c-Met receptor. The treatment with tyrosine kinase inhibitor TKI small molecule Gefitinib (gefitinib, an epidermal growth factor receptor tyrosine kinase inhibitor) would induce apoptosis in HCC827 cells. If HGF was added at the same time under such conditions, the c-Met pathway would be activated, thereby inducing HCC827 to resist to Gefitinib, and inhibiting apoptosis) were adjusted to reach a cell density of 2×10⁴ cells/ml, added to a 96-well cell culture plate at 100 µl/well, and cultured overnight for later use. The antibody to be tested was diluted to 300 nM with 1640 medium, and then subjected to 3-fold dilution in series; HGF was diluted to 300 ng/mL, and Gefitinib was diluted to 0.6 µM. According to the experimental requirements, the diluted antibody at 50 µl/well, HGF at 25 µl/well, and Gefitinib at 25 µl/well were added to the 96 plate with cells, and 1640 medium was supplemented to reach a total volume of 150 µl/well, and cultured for 5 days at 37 °C and 5% carbon dioxide. After 5 days, 100 µl of the medium was removed, then 100 µl/well of Cell titer glo (purchased from Promega) was added, and chemiluminescent signal was collected with an microplate reader.

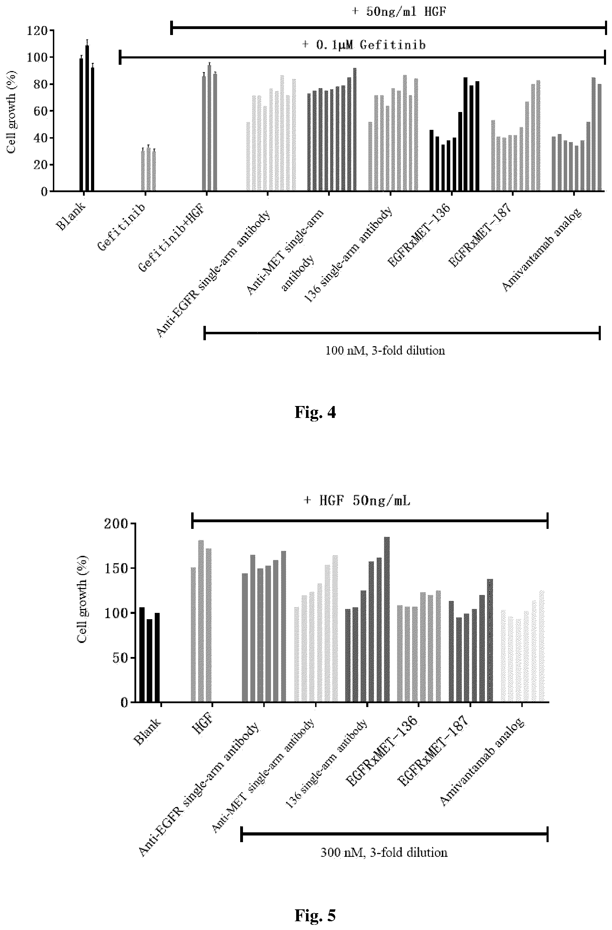

As shown in Fig. 4, the blocking strength of EGFRxc-Met bispecific antibody molecules (EGFRxMET-136 and EGFRxMET-187) was significantly better than that of the anti-c-Met single-arm molecules, indicating that the binding to the EGFR could improve the blocking effect of the bispecific antibody molecules on HGF-c-Met signals.

### Example 4: Anti-EGFRxc-Met bispecific antibody blocking HGF-induced cell proliferation

In this experiment, the blocking effect of the anti-EGFRxc-Met bispecific antibodies on HGF-induced cell proliferation was detected. The expanded H596 cells were adjusted to have a density of 3×10⁴ cells/ml, added to a 96-well cell culture plate at 100 µl/well, and cultured overnight for later use. The antibody to be tested was diluted to 1200 nM with 1640 culture medium, and then subjected to 3-fold dilution; and HGF was diluted to 200 ng/mL. The diluted antibody at 50 µl/well, and HGF at 50 µl/well were added to the 96-well plate with cells according to the experimental requirements, and 1640 culture medium was supplemented to reach a total volume of 200 µl/well, and cultured for 5 days at 37°C and 5% carbon dioxide. After 5 days, 100 µl of the culture medium was removed, then 100 µl/well of Cell titer glo (purchased from Promega) was added, and the chemiluminescent signal was collected with an microplate reader.

As shown in Fig. 5, the blocking strength of the EGFRxc-Met bispecific antibody molecules EGFRxMET-136 and EGFRxMET-187 was significantly better than that of the anti-c-Met single-arm antibody, indicating once again that the binding to EGFR could improve the blocking effect of the bispecific antibody molecules on HGF-c-Met signals.

### Example 5: Anti-EGFRxc-Met bispecific antibody blocking HGF-induced cell migration

HepG2 cells were human liver cancer cells and purchased from ATCC, which were capable of expressing EGFR and c-Met receptors. When HGF was placed in the lower chamber culture medium, it would induce HepG2 cells in the upper chamber to migrate to the lower chamber through the filter membrane. The specific experimental method was as follows: HepG2 cells were expanded to an appropriate density, the cells were digested and detached from the cell culture flask, resuspended to 1×10⁶ cells/ml, and added to the upper chamber of the 24-well cell migration plate (purchased from Corning) at 100 µL per well for later use. The bispecific antibody was diluted to 200 nM with MEM, added to the upper chamber of the 24-well cell migration plate at 100 µL per well, and incubated at 25°C for 60 min. h.HGF (purchased from R&D) was diluted to 50 ng/mL with MEM, added to the lower chamber of the cell migration plate at 500 µL/well, and cultured for 3 days at 37°C and 5% carbon dioxide. The non-migrated cells on the upper chamber membrane were gently wiped with cotton swabs, the cells that migrated to the lower chamber membrane were lysed with Cell titer glo (purchased from Promega), and the chemiluminescent signal was collected with an microplate plate reader.

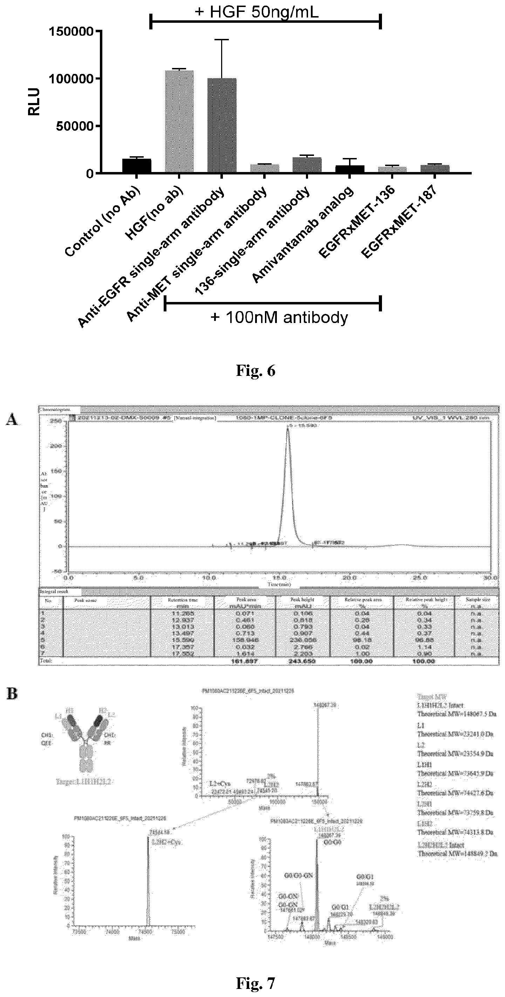

As shown in Fig. 6, the EGFRxc-Met bispecific antibody molecules EGFRxMET-136 and EGFRxMET-187 almost completely blocked the migration of HepG2 cells induced by HGF. As a negative control, the anti-EGFR single-arm antibody prepared based on the control antibody Amivantamab could not block the HGF-induced signal.

### Example 6: Screening of cell lines

In this example, the CH1/CL preference mutation (patent application: WO2021/067404A2) and Knob in hole technology were used to construct EGFRxMET-136 Afu molecules, and the heavy chain variable regions of anti-cMet antibody and anti-EGFR antibody were respectively constructed into the CH1 mutant heavy chain constant regions CH SET1 (SEQ ID NO: 19) and CH SET2 (SEQ ID NO: 20); the light chain variable regions of anti-cMet antibody and anti-EGFR antibody were respectively constructed into the CL mutant light chain constant regions CL SET1 (SEQ ID NO: 21) and CL SET2 (SEQ ID NO: 22). A 1+1 asymmetric anti-EGFRxcMet bispecific antibody cell line was constructed.

### Cell line construction

The vector pCHO2.0-GS-Puro-H1-L1 containing the nucleotide sequences encoding the anti-c-Met antibody heavy chain (SEQ ID NO: 23) and light chain (SEQ ID NO: 24) and the vector pCHO2.0-GS-Puro-H2-L2 containing the nucleotide sequences encoding the anti-EGFR antibody heavy chain (SEQ ID NO: 25) and light chain (SEQ ID NO: 26) were co-transfected into the host cell CHOS-ADP Fut8 KO (afucosylation cell line with gene knockout) by electroporation. The cells were screened by Puromycin and MSX screening pressure to obtain a high-yield minipool, and then a round of limiting dilution and monoclonal identification was performed to obtain a high-yield stable clone cell line. By comparing the growth status, protein expression and physicochemical properties of the expressed protein, the clone cell line 6F5 was finally determined to be a recombinant engineering cell line.

### Cell culture

Using Dynamis AGT Medium as the base medium, the cells were cultured with an inoculation density of (1.0±0.2) ×10⁶ cells/ml. On the 3^{rd}, 5^{th}, 7^{th}, 9^{th}, and 11^{th} days of the culture, 5.0±0.5% (w/w) 7a additional medium relative to the initial culture weight was added in the manner of fed-batch, and 0.5±0.05% (w/w) 7b additional medium relative to the initial culture weight was added in the manner of fed-batch. The dissolved oxygen was set at 40%, and the initial culture temperature was 36.5°C, which was cooled to 33.0°C on the 4^{th} day. According to the detection results of glucose concentration, 300 g/kg of glucose concentrate was supplemented every day to make the glucose concentration in the cell fluid reach 6.0 g/L, except on the day of harvest. The culture was terminated on the 14^{th} day or when the cell viability was less than 80%. During the culture process, Vicell (Beckman) was used to detect the cell density and viability. Starting from the 7^{th} day, Cedex (Roche) was used to detect the antibody production every day. The results were shown in Table 4. The expression of the bispecific antibody reached 2.432 g/L.

**Table 4. Monitoring data of protein production of bispecific antibody expression cell line**

| Culture time (days) | 7 | 8 | 9 | 10 | 11 | 12 | 13 | 14 |
|---|---|---|---|---|---|---|---|---|
| Antibody production (g/L) | 0.853 | 1.095 | 1.358 | 1.573 | 1.865 | 2.037 | 2.158 | 2.432 |

### Quality identification of stable cell line product

The one-step purification method was the same as the purification method of the single-arm antibody in Example 1. The purity of the obtained protein was detected by HPLC. The HPLC method was as follows: mobile phase: 150 mM Na₂HPO₄•12H₂O, pH 7.0. Chromatographic conditions: detection wavelength: 280 nm, column temperature: 25°C, flow rate: 0.5 ml/min, detection time: 30 min, TSKgel G3000SWXL column. The SEC results were shown in Fig. 7A. The purity of the bispecific antibody obtained by the one-step affinity purification was 98.18%.

The pairing of heavy and light chains of the obtained protein was detected by high performance liquid chromatography-mass spectrometry. The instruments used were liquid phase system Vanquish UHPLC (Thermo), mass spectrometer Q Exactive (Thermo) and chromatographic column Waters ACQUITY UPLC BEH C4, 2.1 mm×100 mm. 50 µg of sample was taken, diluted to 25 µl by adding ultrapure water, centrifuged, then 20 µl of the sample was taken and placed in an injection bottle, 5 µl of the sample was injected, and LC-MS was used to analyze the intact molecular weight. The chromatographic conditions: column temperature: 80°C; UV detection wavelength: 280 nm; flow rate: 0.3 mL/min; mobile phase A: aqueous solution (containing 0.1% formic acid); mobile phase B: acetonitrile solution (containing 0.1% formic acid). The mass spectrometry parameters: ESI ion source: ion transfer tube temperature 320°C, voltage 3.8 kV, gas flow rate 36 L/min; mode: positive ion Full MS; resolution: 17500; scanning range: 600 to 4000 m/z. The results were shown in Fig. 7B, and the correct pairing product ratio of the purified bispecific antibody was >98%.

In this example, the afucosylated bispecific antibody Final EGFRxMET-136 (named EGFRxMET-136 Afu) was prepared, and this antibody molecule was used as the final molecule for subsequent in vivo and in vitro activity detection experiments.

### Example 7: Binding activity of anti-EGFRxc-Met bispecific antibody to EGFR and c-Met positive tumor cells

A375 cells were human malignant melanoma cells and purchased from Addexbio, which were capable of low expression of EGFR and c-Met receptors. H292 cells were human lung adenocarcinoma cells and purchased from procell, which were capable of high expression of EGFR and c-Met receptors. HCC827 cells were human non-small cell lung cancer cells and purchased from the Cell Bank of the Chinese Academy of Sciences, which were capable of high expression of EGFR and c-Met receptors. The expanded cells were adjusted to an appropriate cell density, and added to a 96-well flow plate. After centrifugation, the gradiently diluted samples to be tested were added and incubated at 4°C for 1 hour. The cells were washed twice with PBS, then a fluorescent secondary antibody, diluted to an appropriate concentration, was added, incubated at 4°C for 30min, and washed twice with PBS. The cells were resuspended by PBS, and detected on a CytoFlex flow cytometer, and corresponding MFI was calculated.

The results were shown in Fig.s 8A, 8B, and 8C. For the tumor cells with different EGFR and MET expression level, EGFRxc-Met bispecific antibody candidate molecules EGFRxMET-136 Afu and EGFRxMET-187 Afu both showed higher cell binding activity. On the A375, H292 and HCC827 cells, the binding activity EC50 values of EGFRxMET-136 Afu were 1.35nM, 2.34nM, 3.67nM, respectively, and the binding activity EC50 values of EGFRxMET-187 Afu were 1.05nM, 1.46nM, 2.89nM, respectively.

The EC50 values of antibody binding strength were shown in Table 5.

**Table 5: Binding activity of antibodies to tumor cells**

| | A375 EC50(nM) | H292 EC50(nM) | HCC827 EC50(nM) |
|---|---|---|---|
| EGFRxMET-136 Afu | 1.35 | 2.34 | 3.67 |
| EGFRxMET-187 Afu | 1.05 | 1.46 | 2.89 |

### Example 8: Effect of anti-EGFRxc-Met bispecific antibody in inducing ADCC

A375 cells were human malignant melanoma cells and purchased from Addexbio, which were capable of expressing EGFR and c-Met receptors. H1975 cells were human lung adenocarcinoma cells and purchased from Addexbio, which were capable of highly expressing EGFR and c-Met receptors. HCC827 cells were human non-small cell lung cancer cells and purchased from the Cell Bank of the Chinese Academy of Sciences, which were capable of highly expressing EGFR and c-Met receptors. The expanded A375/H1975/HCC827 cells were resuspended in 1640 medium at a cell density of 1.2×10⁶ cells/ml; the CD16a-NFAT-Luc Jurkat reporter gene cells (self-made by BIOTHEUS; CD16a and NFAT-Luc gene sequences were constructed on pCDNA3.1 vector, and then transfected into Jurkat cells, and the CD16a-NFAT-Luc Jurkat reporter gene cells were obtained by antibiotic resistance pressure screening) were resuspended in 1640 medium, and the cell density was adjusted to 6×10⁶ cells/ml; the antibody was diluted to 300nM in 1640 medium, and then subject to 4-fold dilution in series, and 25 ul of the above-mentioned gradiently diluted antibody was added to each well of a 96-well cell culture plate, and 25 ul of the above-mentioned target cells were added to each well, mixed and incubated at room temperature for 30 min, and then 25 ul of the above-mentioned effector cells were added to each well, mixed and incubated at 37°C, 5% CO₂ for 6 h, and then 75 ul of Bio-turbo reagent (purchased from Ruian) was added to each well, mixed, and chemiluminescent signals were collected with a microplate reader.

The results were shown in Fig.s 9A, 9B, and 9C. The candidate EGFRxc-Met bispecific antibody molecules EGFRxMET-136 and EGFRxMET-187 and the control molecule Amivantamab analog all induced ADCC effects and showed dependence on the level of antigen expression (HCC827>H1975>A375). The afucosylated bispecific antibody (EGFRxMET-136 Afu, Amivantamab analog Afu) showed a stronger ADCC effect than the wild-type antibody containing fucose (EGFRxMET-136, Amivantamab analog), and this enhancement was particularly evident in the cells with relatively low antigen expression (A375/H1975).

Using lentiviral transfection method, the plasmid encoding luciferase cDNA (the sequence was from Uniprot, P08659, constructed by General Biol on the pLVX-neu vector) was transfected into A375 cells to construct a luciferase stable transfection cell line (A375-luc). The target cells and PBMC cells were resuspended in 1640 medium and plated into 96-well culture plates at 2×10⁴ and 2×10⁵ cells per well, respectively; the antibody was diluted to 300nM in 1640 medium, then subjected to 3-fold dilution in series, and 50 µl thereof was taken and added to the above 96-well plates, and finally 1640 medium was supplemented to reach a total volume of 150 µl/well. The cells were cultured at 37°C, 5% CO₂ for 48 h, 100 µl of Bio-turbo reagent (purchased from Ruian) was added to each well, mixed well, and then the chemiluminescent signal was collected using an microplate reader.

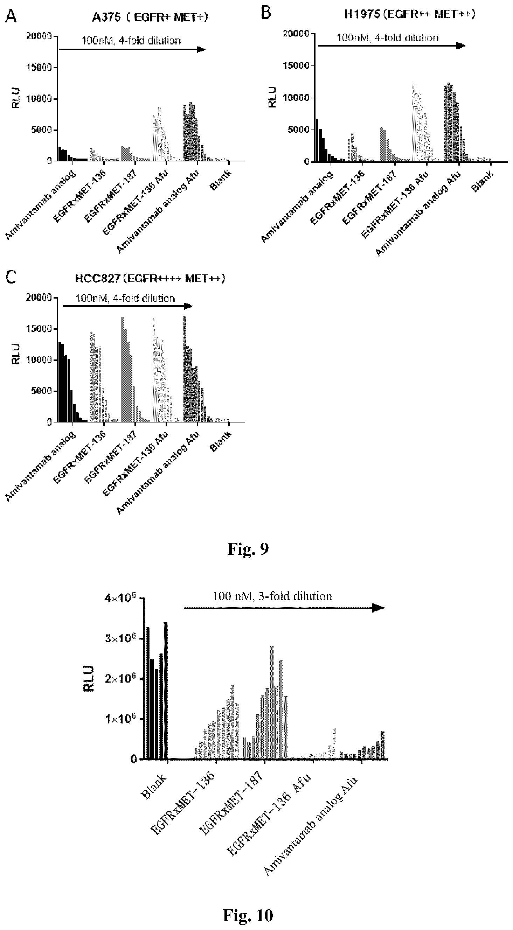

As shown in Fig. 10, the candidate EGFRxc-Met bispecific antibody molecules all induced PBMC to kill target cells. Similarly, the afucosylated bispecific antibody showed a stronger ability to induce PBMC to kill tumor cells as compared to the wild-type antibody containing fucose.

### Example 9: Cell killing of anti-EGFRxc-Met bispecific antibody combined with EGFR small molecule inhibitor

Using lentiviral transfection, cDNAs encoding luciferase (the sequence was from Uniprot, P08659) and human HGF (hepatocyte growth factor) (the sequence was from UniProtKB P14210) were constructed on the pLVX-neu vector by General Biol, and the plasmid was transfected into HCC827 cells to construct a cell line (HCC827-HGF luc) with stable co-expression of luciferase and HGF. Since overexpression of HGF makes HCC827 cells resistant to first-generation EGFR small molecule inhibitors, an experiment of combining antibodies with first-generation small molecules was designed. The expanded luciferase stably transfected cell line (HCC827-HGF Luc) was digested, centrifuged, counted, and resuspended in working medium (10% FBS + RPMI 1640). The cells were adjusted to a density of 3×10⁴/ml, and added to a culture plate at 100 µL per well. PBMC cells were resuscitated from liquid nitrogen, centrifuged, then resuspended in working medium, and added to a culture flask for adherent culture overnight to remove mononuclear cells. After overnight culture, the concentration of suspended immune cells was adjusted to 1.8×10⁵/ml for later use; the antibody was diluted to 800 nM with working medium, and then subjected to 3-fold dilution in series; a solution was prepared with working medium to have the small molecule Gefitinib at a concentration of 0.8 µM, and the small molecule Erlotinib at a concentration of 8 µM; 50 µL of the PBMC cell suspension, 25 µL of the above gradiently diluted antibody, and 25 µL of Gefitinib/Erlotinib solution were added to the corresponding experimental wells on the culture plate with HCC827-HGF luc cells according to experimental requirements, among which, the experimental wells where only some of the components were added were filled with working medium to a final volume of 200 µL per well, after mixing well, the plate was placed and cultured in a 37°C, CO₂ incubator for 48 h; the cell culture plate was taken out and 100 µL of the culture medium supernatant was removed, and then 100 µL of Bio-turbo reagent (purchased from Ruian) was added to each well, and the fluorescence signal value was read using a SpectraMAX microplate reader.

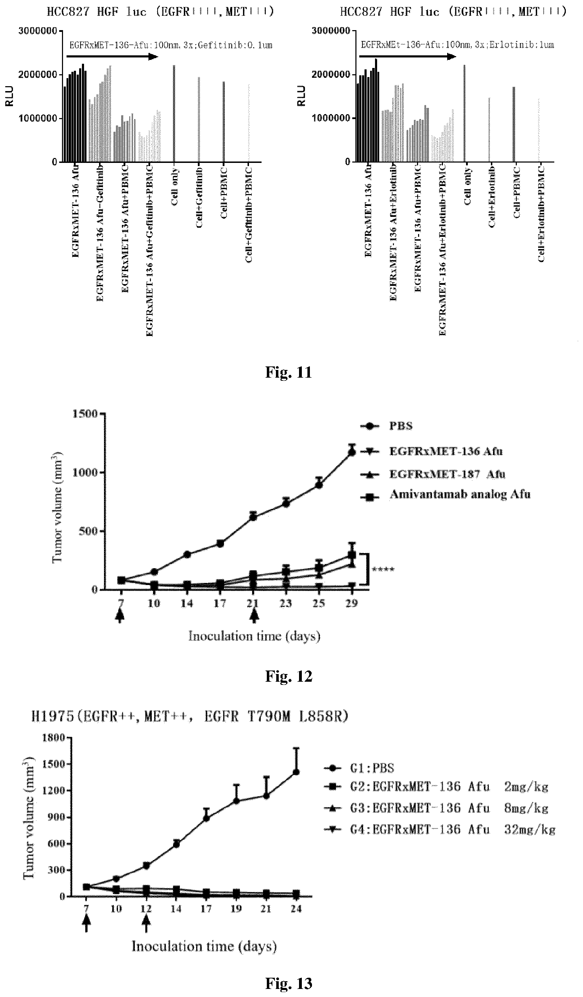

In this example, EGFRxMET-136 Afu molecule was used in combination with the small molecule EGFR inhibitors (Gefitinib on the left, and Erlotinib on the right), and PBMCs were added at the same time to simulate the killing of tumor cells by immune cells. The results were shown in Fig. 11. Without the addition of PBMCs, the EGFRxMET-136 Afu molecule blocked the HGF-MET signaling pathway, relieved the tolerance of HGF-dependent tumor cells to the small molecule inhibitors, and promoted the killing of tumors by the small molecule inhibitors. More importantly, after adding PBMCs, compared with the use of EGFRxMET-136 Afu or small molecule inhibitors alone, the combination thereof showed a synergistic effect and could kill tumor cells more effectively.

### Example 10: Anti-EGFRxc-Met bispecific antibodies inhibiting tumor growth in vivo

In order to study the in vivo efficacy of EGFRxMET-136 Afu and EGFRxMET-187 Afu bispecific molecules, five different animal experimental models were designed in this example for verification.

### Experimental Model 1

In this experiment, CB-17 SCID mice were subcutaneously inoculated with H292 human lung cancer tumor cells to establish a tumor-bearing model and determine the anti-tumor effect of the anti-EGFRxc-Met bispecific antibody. Sufficient H292 cells were cultured and expanded in vitro, and the cells were collected after trypsin digestion. After washing with PBS for 3 times, the cells were counted and inoculated subcutaneously at the right abdomen of female 8-week-old CB-17 SCID mice (purchased from Beijing Weitong Lihua) at an amount of 3×10⁶ cells/mouse. The subcutaneous tumor formation of tumor cells in the mice was observed daily, and the maximum width axis W and the maximum length axis L of the subcutaneous tumor on the right abdomen of each animal were measured with a vernier caliper, and the weight of each mouse was weighed with an electronic balance. The subcutaneous tumor volume on the right abdomen of each mouse was calculated according to the following formula: tumor volume T = 1/2×W×W×L. The mice with too large and too small tumor volumes were eliminated, and the remained mice were divided into 4 groups according to the average tumor volume, with 6 mice in each group. The groups were divided according to the grouping and dosing scheme in Table 6 and the corresponding doses of antibodies were injected.

**Table 6: Experimental scheme for tumor inhibition activity**

| Group | Administered drug | Administration dosage and administration frequency |
|---|---|---|
| Group 1 | PBS | - |
| Group 2 | Amivantamb analog Afu | Day 7: 10mg/kg; Day 21: 2mg/kg |
| Group 3 | EGFRxMET-187 Afu | Day 7: 10mg/kg; Day 21: 2mg/kg |
| Group 4 | EGFRxMET-136 Afu | Day 7: 10mg/kg; Day 21: 2mg/kg |

The tumor volume and weight of mice were measured 2 to 3 times a week. The weight and tumor volume of mice were measured for the last time 29 days after the tumor cell inoculation, and then the mice were euthanized.

The results were shown in Fig. 12. Compared with the PBS group, the three bispecific antibody groups all showed significant inhibitory effects on tumor growth, among which the EGFRxMET-136 Afu bispecific antibody group was significantly more effective than the Amivantamb analog Afu. The tumor (volume) inhibition rate (TGI) corresponding to each group was calculated, among which the TGI of the EGFRxMET-136 Afu molecule group was 97.3%, the TGI of the EGFRxMET-187 Afu molecule group was 81.0%, and the TGI of the Amivantamab analog Afu molecule group was 74.5% (the TGI of EGFRxMET-136 Afu was significantly different from that of the Amivantamab analog Afu).

### Experimental Model 2

In this experiment, CB-17 SCID mice were subcutaneously inoculated with H1975 human lung cancer tumor cells to establish a tumor-bearing model and determine the anti-tumor effect of the anti-EGFRxc-Met bispecific antibody. Sufficient H1975 cells were cultured and expanded in vitro, and the cells were collected after trypsin digestion. After washing 3 times with PBS, the cells were counted, and inoculated subcutaneously at 3×10⁶ cells/mouse into the right abdomen of female 8-week-old CB-17 SCID mice (purchased from Beijing Weitong Lihua). The subcutaneous tumor formation of tumor cells in the mice was observed daily. The maximum width axis W and the maximum length axis L of the subcutaneous tumor on the right abdomen of each animal were measured with a vernier caliper, and the weight of each mouse was weighed with an electronic balance. The subcutaneous tumor volume on the right abdomen of each mouse was calculated according to the following formula: tumor volume T = 1/2×W×W×L. The mice with too large and too small tumor volumes were eliminated, and the remained mice were divided into 4 groups according to the average tumor volume, with 6 mice in each group. The mice were divided into groups according to the grouping and dosing scheme in Table 7 and injected with the corresponding doses of antibodies.

**Table 7: Experimental scheme for tumor inhibition activity**

| Group | Administered drug | Administration dosage and administration frequency |
|---|---|---|
| Group 1 | PBS | - |
| Group 2 | EGFRxMET-136 Afu | Day 7: 2mg/kg; Day 21: 2mg/kg |
| Group 3 | EGFRxMET-136 Afu | Day 7: 8mg/kg; Day 21: 8mg/kg |
| Group 4 | EGFRxMET-136 Afu | Day 7: 32mg/kg; Day 21: 32mg/kg |

The tumor volume and body weight of mice were measured 2 to 3 times a week. The body weight and tumor volume of mice were measured for the last time 24 days after the tumor cell inoculation, and then the mice were euthanized.

The results were shown in Fig. 13. Compared with the PBS group, the three bispecific antibody groups with different doses (2 mg/kg, 8 mg/kg, 32 mg/kg) all showed significant inhibitory effects on tumor growth. Among them, the TGI of the 2 mg/kg treatment group was 97.4%, the TGI of the 8 mg/kg treatment group was 99.3%, and the TGI of the 32 mg/kg treatment group was 99.4%. It could be seen that the bispecific antibodies of the present application almost completely inhibited tumor growth, and the tumor inhibition rate had reached more than 99%.

### Experimental Model 3

In this experiment, CB-17 SCID mice were subcutaneously inoculated with H292 human lung cancer tumor cells to establish a tumor-bearing model and compare the anti-tumor effects of different doses of anti-EGFRxc-Met bispecific antibodies. The mouse model establishment process was the same as Experimental Model 1. The groups were divided according to the grouping and administration scheme in Table 8 and the corresponding doses of antibodies were injected.

**Table 8. Experimental scheme for tumor inhibition activity**

| Group | Administered drug | Administration dosage and administration frequency |
|---|---|---|
| Group 1 | PBS | - |
| Group 2 | EGFRxMET-136 Afu | Day 14; Day 18; Day 21: 4 mg/kg/time |
| Group 3 | EGFRxMET-136 Afu | Day 14; Day 18; Day 21: 16 mg/kg/time |

The tumor volume and weight of mice were measured 2 to 3 times a week. The weight and tumor volume of mice were measured for the last time 30 days after the tumor cell inoculation, and then the mice were euthanized.

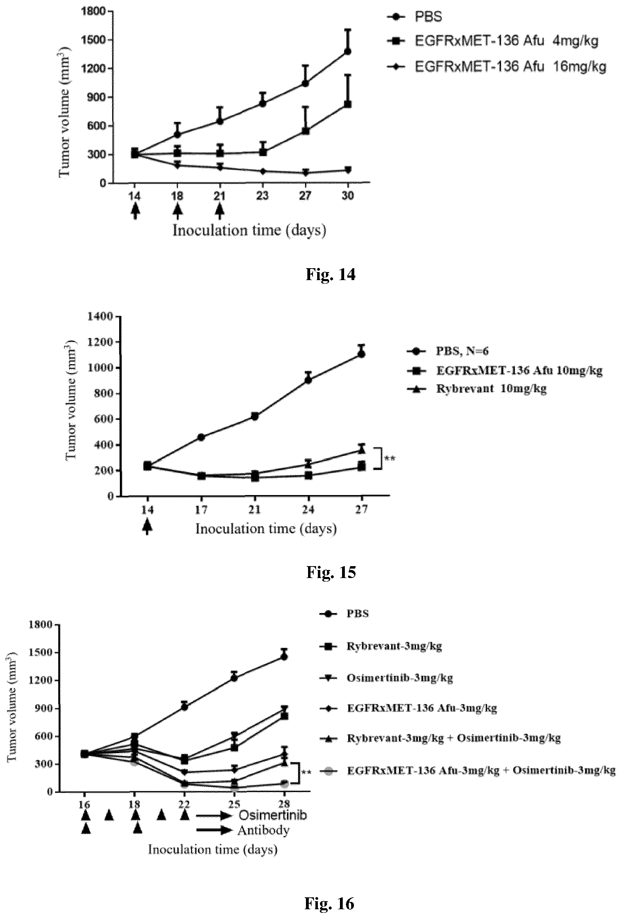

The results were shown in Fig. 14. Compared with the PBS group, the EGFRxMET-136 Afu molecule showed a significant effect of inhibiting tumor growth. Compared with the 4 mg/kg group, the 16 mg/kg group had a stronger tumor inhibition effect, among which the EGFRxMET-136 Afu molecule 4 mg/kg group showed TGI = 40.3%, and the EGFRxMET-136 Afu molecule 16 mg/kg group showed TGI = 90.5%.

### Experimental Model 4

In this experiment, CB-17 SCID mice were subcutaneously inoculated with H292 human lung cancer tumor cells to establish a tumor-bearing model, and the anti-EGFRxc-Met bispecific antibody was compared with the commercially available EGFRxc-Met bispecific antibody Rybrevant (purchased from Johnson & Johnson, batch number: LHS3G02) (this antibody had been published at various oncology conferences and was also known as JNJ-6372) in terms of anti-tumor effect. The mouse model establishment process was the same as that of Experimental Model 1. The groups were divided according to the grouping and dosing scheme in Table 9 and the corresponding doses of antibodies were injected.

**Table 9. Experimental scheme for tumor inhibition activity**

| Group | Administered drug | Administration dosage and administration frequency |
|---|---|---|
| Group 1 | PBS | - |
| Group 2 | EGFRxMET-136 Afu | Day 14: 10 mg/kg |
| Group 4 | Rybrevant | Day 14: 10 mg/kg |

The tumor volume and body weight of the mice were measured 2 to 3 times a week. The weight and tumor volume of the mice were measured for the last time 27 days after the inoculation of tumor cells, and the mice were euthanized.

The results were shown in Fig. 15. Compared with the PBS group, the EGFRxMET-136 Afu group had a significantly stronger tumor inhibition effect than that of the Rybrevant group. Among them, the TGI of the EGFRxMET-136 Afu molecule group was 79.9%, and the TGI of the Rybrevant molecule group was 67.9% (the TGI results of the two were significantly different).

### Experimental Model 5

In this experiment, the in vivo efficacy of the bispecific antibody of the present application in combination with an small molecule EGFR inhibitor (e.g., Osimertinib) was detected. Using the lentiviral transfection method, the cDNA encoding human HGF (hepatocyte growth factor) (the sequence was from UniProtKB P14210) was constructed on the pLVX-neu vector by General Biol, and the plasmid was transfected into H1975 cells to construct a cell line (H975-HGF) with stable expression of HGF. In this experiment, CB-17 SCID mice were subcutaneously inoculated with H1975-HGF human lung cancer cells to establish a tumor-bearing model and determine the anti-tumor effect of the anti-EGFRxc-Met bispecific antibody. The model construction process was the same as that of Experimental Model 2. The mice were divided into groups according to the grouping and dosing scheme in Table 10 and the corresponding doses of antibodies were injected.

**Table 10. Experimental scheme for tumor inhibition activity**

| Group | Administered drug | Administration dosage and administration frequency |
|---|---|---|
| Group 1 | PBS | - |
| Group 2 | Rybrevant | Day 16 and Day 18: 3mg/kg |
| Group 3 | Osimertinib | Days 16 to 22: 3mg/kg/day |
| Group 4 | EGFRxMET-136 Afu | Day 16 and Day 18: 3mg/kg |
| Group 5 | Rybrevant+Osimertinib | Rybrevant: Day 16 and Day 18: 3mg/kg; |
| | | Osimertinib: Days 16 to 22: 3mg/kg/day |
| Group 6 | EGFRxMET-136 Afu+ Osimertinib | EGFRxMET-136 Afu: Day 16 and Day 18: 3mg/kg; |
| | | Osimertinib: Days 16 to 22: 3mg/kg/day |

The tumor volume and body weight of mice were measured 2 to 3 times a week. The body weight and tumor volume of mice were measured for the last time 28 days after the tumor cell inoculation, and then the mice were euthanized.

The results were shown in Fig. 16. Compared with the PBS group, each experimental group showed significant anti-tumor effects; the EGFRxMET-136 Afu combined with a small molecule inhibitor had a stronger anti-tumor activity than the group of a bispecific antibody or a small molecule alone, and significantly stronger than other treatment groups; at the same time, the group of EGFRxMET-136 Afu combined with a small molecule had stronger tumor inhibition effect than the group of Rybrevant combined with a small molecule. Among them, the Osimertinib treatment group showed TGI = 38.9%, the Rybrevant treatment group showed TGI = 44.1%, the EGFRxMET-136 Afu treatment group showed TGI = 72.3%, the group of Rybrevant combined with a small molecule showed TGI = 78.7%, and the group of EGFRxMET-136 Afu combined with a small molecule showed TGI = 89.8%.

Although the specific embodiments of the present invention have been described in detail, those skilled in the art will understand that various modifications and changes can be made to the details based on all the teachings that have been published, and these changes are within the scope of protection of the present invention. The entirety of the present invention is given by the appended claims and any equivalents thereof.

## Claims

1. An antibody or antigen-binding fragment thereof capable of specifically binding to c-Met, the antibody or antigen-binding fragment thereof comprising:
(a) a heavy chain variable region (VH) comprising the following 3 complementarity determining regions (CDRs):
(i) VH CDR1, which is composed of the sequence as set forth in SEQ ID NO: 27 or 33, or a sequence having a substitution, deletion or addition of one or several amino acids (e.g., a substitution, deletion or addition of 1, 2 or 3 amino acids) as compared thereto,
(ii) VH CDR2, which is composed of the sequence as set forth in SEQ ID NO: 28 or 34, or a sequence having a substitution, deletion or addition of one or several amino acids (e.g., a substitution, deletion or addition of 1, 2 or 3 amino acids) as compared thereto, and
(iii) VH CDR3, which is composed of the sequence as set forth in any one of SEQ ID NO: 29 or 35, or a sequence having a substitution, deletion or addition of one or several amino acids (e.g., a substitution, deletion or addition of 1, 2 or 3 amino acids) as compared thereto;
and/or,
(b) a light chain variable region (VL) comprising the following 3 complementarity determining regions (CDRs):
(iv) VL CDR1, which is composed of the following sequence: SEQ ID NO: 30 or 36, or a sequence having a substitution, deletion or addition of one or several amino acids (e.g., a substitution, deletion or addition of 1, 2 or 3 amino acids) as compared thereto,
(v) VL CDR2, which is composed of the following sequence: SEQ ID NO: 31 or 37, or a sequence having a substitution, deletion or addition of one or several amino acids (e.g., a substitution, deletion or addition of 1, 2 or 3 amino acids) as compared thereto, and
(vi) VL CDR3, which is composed of the following sequence: SEQ ID NO: 32 or 38, or a sequence having a substitution, deletion or addition of one or several amino acids (e.g., a substitution, deletion or addition of 1, 2 or 3 amino acids) as compared thereto;
preferably, the substitution described in any one of (i) to (vi) is a conservative substitution;
preferably, the CDR described in any one of (i) to (vi) are defined according to the Kabat, IMGT or Chothia numbering system;
preferably, the CDR described in any one of (i) to (vi) are defined according to the IMGT numbering system.

2. The antibody or antigen-binding fragment thereof according to claim 1, comprising:
(1) the following 3 heavy chain CDRs: VH CDR1 as set forth in SEQ ID NO: 27, VH CDR2 as set forth in SEQ ID NO: 28, VH CDR3 as set forth in SEQ ID NO: 29; and/or, the following 3 light chain CDRs: VL CDR1 as set forth in SEQ ID NO: 30, VL CDR2 as set forth in SEQ ID NO: 31, VL CDR3 as set forth in SEQ ID NO: 32; or
(2) the following 3 heavy chain CDRs: VH CDR1 as set forth in SEQ ID NO: 33, VH CDR2 as set forth in SEQ ID NO: 34, VH CDR3 as set forth in SEQ ID NO: 35; and/or, the following 3 light chain CDRs: VL CDR1 as set forth in SEQ ID NO: 36, VL CDR2 as set forth in SEQ ID NO: 37, VL CDR3 as set forth in SEQ ID NO: 38;
preferably, the antibody or antigen-binding fragment thereof further comprises a framework region of a human immunoglobulin.

3. The antibody or antigen-binding fragment thereof according to claim 1 or 2, wherein the antibody or antigen-binding fragment thereof comprises:
(a) a heavy chain variable region (VH), which comprises an amino acid sequence selected from the following:
(i) the sequence as set forth in SEQ ID NO: 9 or 13;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 9 or 13; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 9 or 13;
and/or
(b) a light chain variable region (VL), which comprises an amino acid sequence selected from the following:
(iv) the sequence as set forth in SEQ ID NO: 11 or 15;
(v) a sequence having a substitution, deletion or addition of one or several amino acids (e.g., a substitution, deletion or addition of 1, 2, 3, 4 or 5 amino acids) as compared to the sequence as set forth in SEQ ID NO: 11 or 15; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 11 or 15;
preferably, the substitution described in (ii) or (v) is a conservative substitution;
preferably, the antibody or antigen-binding fragment thereof comprises:
(1) a VH having a sequence as set forth in SEQ ID NO: 9 and a VL having a sequence as set forth in SEQ ID NO: 11;
(2) a VH having a sequence as set forth in SEQ ID NO: 13 and a VL having a sequence as set forth in SEQ ID NO: 15.

4. The antibody or antigen-binding fragment thereof according to any one of claims 1 to 3, wherein it further comprises a constant region derived from a human immunoglobulin;
preferably, the heavy chain of the antibody or antigen-binding fragment thereof comprises a heavy chain constant region derived from a human immunoglobulin (e.g., IgG1, IgG2, IgG3 or IgG4);
preferably, the heavy chain constant region has a sequence as set forth in SEQ ID NO: 19, 20, 39 or 40;
preferably, the light chain of the antibody or antigen-binding fragment thereof comprises a light chain constant region derived from a human immunoglobulin (e.g., κ or λ);
preferably, the light chain constant region has a sequence as set forth in SEQ ID NO: 21, 22 or 41;
preferably, the antibody or antigen-binding fragment thereof has ADCC activity;
preferably, the antibody or antigen-binding fragment thereof comprises a mutated or chemically modified Fc region;
preferably, the antibody or antigen-binding fragment thereof comprises an Fc region having a LALA mutation and/or a knob-into-hole modification;
preferably, the Fc region has an amino sequence as set forth in SEQ ID NO: 17 or 18;
preferably, the antibody or antigen-binding fragment thereof is hypofucosylated or afucosylated;
preferably, the antibody or antigen-binding fragment thereof comprises:
(1) a heavy chain having a sequence as set forth in SEQ ID NO: 10 and a light chain having a sequence as set forth in SEQ ID NO: 12;
(2) a heavy chain having a sequence as set forth in SEQ ID NO: 14 and a light chain having a sequence as set forth in SEQ ID NO: 16; or
(3) a heavy chain having a sequence as set forth in SEQ ID NO: 23 and a light chain having a sequence as set forth in SEQ ID NO: 24.

5. The antibody or antigen-binding fragment thereof according to any one of claims 1 to 4, wherein the antigen-binding fragment is selected from the group consisting of Fab, Fab', (Fab')², Fv, disulfide-bonded Fv, scFv, diabody and single domain antibody (sdAb); and/or the antibody is a murine antibody, a chimeric antibody, a humanized antibody, or a multispecific antibody.

6. An isolated nucleic acid molecule, which encodes the antibody or antigen-binding fragment thereof according to any one of claims 1 to 5.

7. A vector, which comprises the nucleic acid molecule according to claim 6; preferably, the vector is a cloning vector or an expression vector.

8. A host cell, which comprises the nucleic acid molecule according to claim 6 or the vector according to claim 7;
preferably, the host cell is a mammalian cell;
preferably, the host cell has low or no fucosylation activity, for example, the host cell is selected from mammalian cells (e.g., CHO cells) lacking expression of a gene encoding a fucosyltransferase.

9. A method for preparing the antibody or antigen-binding fragment thereof according to any one of claims 1 to 5, comprising culturing the host cell according to claim 8 under a condition that allows expression of the antibody or antigen-binding fragment thereof, and recovering the antibody or antigen-binding fragment thereof from a culture of the cultured host cell;
preferably, the host cell has low or no fucosylation activity, for example, the host cell is selected from mammalian cells (e.g., CHO cells) lacking expression of a gene encoding a fucosyltransferase.

10. A multispecific molecule, which comprises the antibody or antigen-binding fragment thereof according to any one of claims 1 to 5;
preferably, the multispecific molecule specifically binds to c-Met and additionally specifically binds to one or more other targets;
preferably, the multispecific molecule is a bispecific molecule;
preferably, the bispecific molecule further comprises a molecule having a second binding specificity for a second target (e.g., a second antibody);
preferably, the second target is an epidermal growth factor receptor (EGFR), and the second antibody is an anti-EGFR antibody or an antigen-binding fragment thereof.

11. The multispecific molecule according to claim 10, wherein the bispecific molecule has been modified by glycosylation so as to have a lower number of fucose than the same bispecific molecule that has not been modified by glycosylation;
preferably, the anti-EGFR antibody or antigen-binding fragment thereof is hypofucosylated or afucosylated;
preferably, the antibody or antigen-binding fragment thereof according to any one of claims 1 to 5 is hypofucosylated or afucosylated;
preferably, the second antibody comprises: the following 3 heavy chain CDRs: VH CDR1 as set forth in SEQ ID NO: 46, VH CDR2 as set forth in SEQ ID NO: 47, VH CDR3 as set forth in SEQ ID NO: 48; and, the following 3 light chain CDRs: VL CDR1 as set forth in SEQ ID NO: 49, VL CDR2 as set forth in SEQ ID NO: 50, VL CDR3 as set forth in SEQ ID NO: 51;
preferably, the second antibody comprises:
(1) a VH having a sequence as set forth in SEQ ID NO: 1 and a VL having a sequence as set forth in SEQ ID NO: 3; or
(2) a VH having a sequence as set forth in SEQ ID NO: 52 and a VL having a sequence as set forth in SEQ ID NO: 53;
preferably, the second antibody comprises:
(1) a heavy chain having a sequence as set forth in SEQ ID NO: 2 and a light chain having a sequence as set forth in SEQ ID NO: 4; or
(2) a heavy chain having a sequence as set forth in SEQ ID NO: 25 and a light chain having a sequence as set forth in SEQ ID NO: 26;
preferably, the bispecific molecule comprises:
(1) a first antibody comprising VH CDRs 1-3 as set forth in SEQ ID NO: 27-29 and VL CDRs 1-3 as set forth in SEQ ID NO: 30-32; and, a second antibody comprising VH CDRs 1-3 as set forth in SEQ ID NO: 46-48 and VL CDRs 1-3 as set forth in SEQ ID NO: 49-51; or
(2) a first antibody comprising VH CDRs 1-3 as set forth in SEQ ID NO: 33-35 and VL CDRs 1-3 as set forth in SEQ ID NO: 36-38; and, a second antibody comprising VH CDRs 1-3 as set forth in SEQ ID NO: 46-48 and VL CDRs 1-3 as set forth in SEQ ID NO: 49-51;
preferably, the multispecific molecule comprises:
(1) a first antibody comprising VH with a sequence as set forth in SEQ ID NO: 9 and VL with a sequence as set forth in SEQ ID NO: 11; and, a second antibody comprising VH with a sequence as set forth in SEQ ID NO: 1 and VL with a sequence as set forth in SEQ ID NO: 3;
(2) a first antibody comprising VH with a sequence as set forth in SEQ ID NO: 9 and VL with a sequence as set forth in SEQ ID NO: 11; and, a second antibody comprising VH with a sequence as set forth in SEQ ID NO: 52 and VL with a sequence as set forth in SEQ ID NO: 53;
(3) a first antibody comprising VH with a sequence as set forth in SEQ ID NO: 13 and VL with a sequence as set forth in SEQ ID NO: 15; and, a second antibody comprising VH with a sequence as set forth in SEQ ID NO: 1 and VL with a sequence as set forth in SEQ ID NO: 3; or
(4) a first antibody comprising VH with a sequence as set forth in SEQ ID NO: 13 and VL with a sequence as set forth in SEQ ID NO: 15; and, a second antibody comprising VH with a sequence as set forth in SEQ ID NO: 52 and VL with a sequence as set forth in SEQ ID NO: 53;
preferably, the multispecific molecule comprises:
(1) a first antibody comprising a heavy chain with a sequence as set forth in SEQ ID NO: 10 and a light chain with a sequence as set forth in SEQ ID NO: 12; and, a second antibody comprising a heavy chain with a sequence as set forth in SEQ ID NO: 25 and a light chain with a sequence as set forth in SEQ ID NO: 26;
(2) a first antibody comprising a heavy chain with a sequence as set forth in SEQ ID NO: 10 and a light chain with a sequence as set forth in SEQ ID NO: 12; and, a second antibody comprising a heavy chain with a sequence as set forth in SEQ ID NO: 2 and a light chain with a sequence as set forth in SEQ ID NO: 4;
(3) a first antibody comprising a heavy chain with a sequence as set forth in SEQ ID NO: 14 and a light chain with a sequence as set forth in SEQ ID NO: 16; and, a second antibody comprising a heavy chain with a sequence as set forth in SEQ ID NO: 25 and a light chain with a sequence as set forth in SEQ ID NO: 26;
(4) a first antibody comprising a heavy chain with a sequence as set forth in SEQ ID NO: 14 and a light chain with a sequence as set forth in SEQ ID NO: 16; and, a second antibody comprising a heavy chain with a sequence as set forth in SEQ ID NO: 2 and a light chain with a sequence as set forth in SEQ ID NO: 4; or
(5) a first antibody comprising a heavy chain with a sequence as set forth in SEQ ID NO: 23 and a light chain with a sequence as set forth in SEQ ID NO: 24; and, a second antibody comprising a heavy chain with a sequence as set forth in SEQ ID NO: 25 and a light chain with a sequence as set forth in SEQ ID NO: 26.

12. A method for preparing the multispecific antibody according to claim 10 or 11, comprising obtaining the antibody or antigen-binding fragment thereof according to any one of claims 1 to 5 by the method according to claim 9, and contacting it with an anti-EGFR antibody or antigen-binding fragment thereof; optionally, contacting it with a reducing agent (e.g., DTT).

13. An immunoconjugate, comprising the antibody or antigen-binding fragment thereof according to any one of claims 1 to 5 or the multispecific molecule according to claim 10 or 11, and a therapeutic agent linked to the antibody or antigen-binding fragment thereof or the multispecific molecule;
preferably, the therapeutic agent is selected from cytotoxic agents;
preferably, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof;
preferably, the immunoconjugate is an antibody-drug conjugate (ADC).

14. A pharmaceutical composition, comprising the antibody or antigen-binding fragment thereof according to any one of claims 1 to 5, or the multispecific molecule according to claim 10 or 11, and a pharmaceutically acceptable carrier and/or excipient;
preferably, the pharmaceutical composition further comprises an additional pharmaceutically active agent;
preferably, the pharmaceutical composition further comprises an EGFR inhibitor;
preferably, the EGFR inhibitor is selected from the group consisting of erlotinib, gefitinib, osimertinib, or any combination thereof; more preferably, the EGFR inhibitor and the antibody or antigen-binding fragment thereof, or the multispecific molecule are respectively contained in different preparations as active components and are administered simultaneously or at different times; preferably, the EGFR inhibitor is osimertinib;
preferably, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus.

15. Use of the antibody or antigen-binding fragment thereof according to any one of claims 1 to 5 or the multispecific molecule according to claim 10 or 11 in combination with an EGFR inhibitor in the manufacture of a medicament;
preferably, the EGFR inhibitor is selected from the group consisting of erlotinib, gefitinib, osimertinib, or any combination thereof; more preferably, the EGFR inhibitor is osimertinib;
preferably, the medicament is used for:
(1) increasing immune cell activity in vitro or in vivo in a subject;
(2) enhancing an immune response in a subject;
(3) preventing and/or treating a tumor in a subject; or
(4) preventing and/or treating an infection in a subject;
preferably, the tumor expresses c-Met;
preferably, the tumor involves a tumor cell expressing c-Met; preferably, the c-Met is expressed on the surface of the tumor cell;
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoides, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-related diffuse large B-cell lymphoma (DLBCL), plasmablastic lymphoma, extranodal NK/T-cell lymphoma, nasopharyngeal carcinoma and HHV8-related primary effusion lymphoma, Hodgkin's lymphoma, central nervous system (CNS) tumors, such as primary CNS lymphoma, spinal axis tumor, brainstem glioma;
preferably, the infection is selected from the group consisting of viral infection, bacterial infection, fungal infection and parasitic infection;
preferably, the subject is a mammal, such as a human, a cynomolgus monkey or a mouse.

16. A kit, comprising the antibody or antigen-binding fragment thereof according to any one of claims 1 to 5;
preferably, the antibody or antigen-binding fragment thereof comprises a detectable label, such as an enzyme (e.g., horseradish peroxidase), a radionuclide, a fluorescent dye, a luminescent substance (e.g., a chemiluminescent substance) or biotin;
preferably, the kit further comprises a second antibody, which specifically recognizes an anti-EGFR antibody or antigen-binding fragment thereof;
preferably, the second antibody further comprises a detectable label, such as an enzyme (e.g., horseradish peroxidase), a radionuclide, a fluorescent dye, a luminescent substance (e.g., a chemiluminescent substance) or biotin;
preferably, the anti-EGFR antibody or antigen-binding fragment thereof is hypofucosylated or afucosylated;
preferably, the antibody or antigen-binding fragment thereof according to any one of claims 1 to 5 is hypofucosylated or afucosylated.

17. A chimeric antigen receptor, comprising an antigen-binding domain of the antibody or antigen binding fragment thereof according to any one of claims 1 to 5;
preferably, the antigen-binding domain comprises a heavy chain variable region and a light chain variable region of the antibody or antigen binding fragment thereof according to any one of claims 1 to 5;
preferably, the chimeric antigen receptor is expressed by an immune effector cell (e.g., a T cell).

18. A method for inhibiting the growth of a tumor cell expressing c-Met and/or killing the tumor cell, comprising contacting the tumor cell with an effective amount of the antibody or antigen binding fragment thereof according to any one of claims 1 to 5, or the multispecific molecule according to claim 10 or 11, or the immunoconjugate according to claim 13, or the pharmaceutical composition according to claim 14, or the chimeric antigen receptor according to claim 16.

19. Use of the antibody or antigen-binding fragment thereof according to any one of claims 1 to 5, or the multispecific molecule according to claim 10 or 11, or the immunoconjugate according to claim 13, or the pharmaceutical composition according to claim 14, or the chimeric antigen receptor according to claim 16 in the manufacture of a medicament, wherein the medicament is used for:
(1) increasing immune cell activity in vitro or in vivo in a subject;
(2) enhancing an immune response in a subject;
(3) preventing and/or treating a tumor in a subject; or
(4) preventing and/or treating an infection in a subject;
preferably, the tumor expresses c-Met;
preferably, the tumor involves a tumor cell expressing c-Met; preferably, the c-Met is expressed on the surface of the tumor cell;
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoides, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-related diffuse large B-cell lymphoma (DLBCL), plasmablastic lymphoma, extranodal NK/T-cell lymphoma, nasopharyngeal carcinoma and HHV8-related primary effusion lymphoma, Hodgkin's lymphoma, central nervous system (CNS) tumors, such as primary CNS lymphoma, spinal axis tumor, brainstem glioma;
preferably, the infection is selected from the group consisting of viral infection, bacterial infection, fungal infection and parasitic infection;
preferably, the subject is a mammal, such as a human, a cynomolgus monkey or a mouse.

20. Use of the antibody or antigen-binding fragment thereof according to any one of claims 1 to 5 in the manufacture of a kit, wherein the kit is used for determining whether a tumor can be treated by an anti-tumor therapy targeting c-Met;
(1) contacting a sample containing the tumor cell with the antibody or antigen-binding fragment thereof according to any one of claims 1 to 5;
(2) detecting the formation of a complex comprising the antibody or antigen-binding fragment thereof and c-Met;
preferably, the antibody or antigen-binding fragment thereof comprises a detectable label;
preferably, the c-Met is c-Met of a mammalian (e.g., a human, a monkey);
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-related diffuse large B-cell lymphoma (DLBCL), plasmablastic lymphoma, extranodal NK/T-cell lymphoma, nasopharyngeal carcinoma and HHV8-related primary effusion lymphoma, Hodgkin's lymphoma, central nervous system (CNS) tumors, such as primary CNS lymphoma, spinal axis tumor, brainstem glioma.
e.
